# EUROPEAN PATENT SPECIFICATION

(11) **EP 4 326 274 B1**
(45) Date of publication and mention of the grant of the patent: **14.01.2026**
(21) Application number: 22792256.4
(22) Date of filing: 18.04.2022
(51) Int. Cl.: C07D 498/10, A61P 3/10, A61P 7/02, A61P 19/02, A61P 27/02, A61P 29/00, A61K 31/537, A61K 45/06

(54) **PLASMA KALLIKREIN INHIBITORS**
PLASMAKALLIKREINHEMMER
INHIBITEURS DE LA KALLIKRÉINE PLASMATIQUE

(30) Priority: 22.04.2021 US 202163178265 P
(43) Date of publication of application: 28.02.2024
(73) Proprietor: Merck Sharp & Dohme LLC, Rahway, New Jersey 07065 (US)
(72) Inventor: CHENG, Alan, C., San Francisco, California 94080 (US); GAO, Ying-Duo, Middletown, New Jersey 07748 (US); YANG, Song, San Francisco, California 94080 (US); MANDAL, Mihir, Kenilworth, New Jersey 07033 (US); HE, Jiafang, Kenilworth, New Jersey 07033 (US); LOPEZ, Jovan Alexander, New Haven, Connecticut 06511 (US); TIAN, Maoqun, Forster, California 94404 (US)
(74) Representative: Merck Sharp & Dohme LLC
(86) International application number: PCT/US2022/025159
(87) International publication number: WO 2022/225828

(56) References cited:
- WO-A1-2017/074833
- WO-A1-2022/109161
- US-A1- 2005 282 858
- US-A1- 2017 044 183

## Description

### BACKGROUND OF THE INVENTION

Plasma kallikrein is a zymogen of a trypsin-like serine protease and is present in plasma. The gene structure is similar to that of factor XI. Overall, the amino acid sequence of plasma kallikrein has 58% homology to factor XI. Proteolytic activation by factor XIIa at an internal I389-R390 bond yields a heavy chain (371 amino acids) and a light chain (248 amino acids). The active site of plasma kallikrein is contained in the light chain. The light chain of plasma kallikrein reacts with protease inhibitors, including alpha 2 macroglobulin and Cl-inhibitor. Interestingly, heparin significantly accelerates the inhibition of plasma kallikrein by antithrombin III in the presence of high molecular weight kininogen (HMWK). In blood, the majority of plasma kallikrein circulates in complex with HMWK. Plasma kallikrein cleaves HMWK to liberate bradykinin. Bradykinin release results in increase of vascular permeability and vasodilation (for review, Coleman, R., "Contact Activation Pathway", Hemostasis and Thrombosis, pp. 103-122, Lippincott Williams & Wilkins (2001); Schmaier A.H., "Contact Activation", Thrombosis and Hemorrhage, pp. 105-128 (1998)).

Patients presenting genetic deficiency on C1-inhibitor suffer from hereditary angioedema (HAE), a lifelong disease that results in intermittent swelling throughout the body, including the hands, feet, face, throat, genitals and gastrointestinal tract. Analysis of blisters arising from acute episodes have been shown to contain high levels of plasma kallikrein, and treatment with a protein-based reversible plasma kallikrein inhibitor, Ecallantide (Kalbitor), has been approved by the FDA for the treatment of acute attacks of HAE (Schneider, L, et al., J.Allergy Clin.Immunol., 120: p.416 (2007)). Recently, an oral plasma kallikrein inhibitor, Berotralstat, gained FDA approval for the prevention of HAE attacks (Zuraw, B., et al., J. Allergy Clin. Immunol.(2020).

Additionally, the plasma kallikrein-kinin system is abnormally abundant in patients diagnosed with advanced diabetic macular edema (DME). Recent publications have shown that plasma kallikrein contributes to observed retinal vascular leakage and dysfunction in diabetic rodent models (A. Clermont, et al., Diabetes, 60:1590 (2011)), and that treatment with a small molecule plasma kallikrein inhibitor ameliorated the observed retinal vascular permeability and other abnormalities related to retinal blood flow.

It would be desirable in the art to develop plasma kallikrein inhibitors having utility to treat a wide range of disorders, including hereditary angioedema, diabetic macular edema and diabetic retinopathy. US2017/044183 and WO2017/074833 describe other plasma kallikrein inhibitor compounds.

### SUMMARY OF THE INVENTION

The present invention relates to compounds of Formula I: 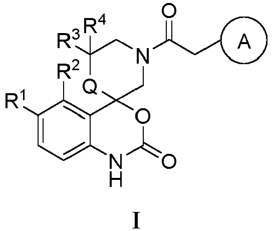 and pharmaceutically acceptable salts thereof. The compounds of Formula I are inhibitors of plasma kallikrein, and as such may be useful in the treatment, inhibition or amelioration of one or more disease states that could benefit from inhibition of plasma kallikrein, including hereditary angioedema, uveitis, posterior uveitis, wet age-related macular degeneration, diabetic macular edema, diabetic retinopathy and retinal vein occlusion. The compounds of this invention could further be used in combination with other therapeutically effective agents, including but not limited to, other drugs useful for the treatment of hereditary angioedema, uveitis, posterior uveitis, wet age-related macular degeneration, diabetic macular edema, diabetic retinopathy and retinal vein occlusion. The invention furthermore relates to processes for preparing compounds of Formula I, and pharmaceutical compositions which comprise compounds of Formula I and pharmaceutically acceptable salts thereof.

### DETAILED DESCRIPTION OF THE INVENTION

The present invention relates to compounds of Formula I: 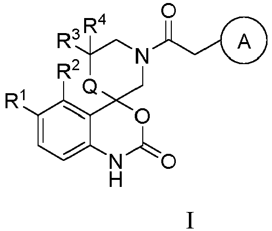 wherein 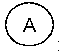 is 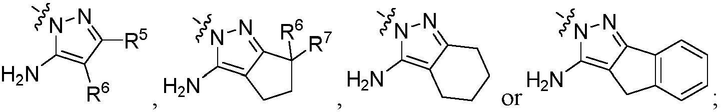
Q is -CH₂- or absent;
R¹ is selected from the group consisting of hydrogen, halo, hydroxy and C₁₋₆ alkyl;
R² is selected from the group consisting of hydrogen, halo, hydroxy and C₁₋₆ alkyl;
R³ is selected from the group consisting of hydrogen, halo, hydroxy and C₁₋₆ alkyl;
R⁴ is selected from the group consisting of hydrogen, halo, hydroxy and C₁₋₆ alkyl;
R⁵ is selected from the group consisting of
   (a) phenyl, which is optionally substituted with one to three substituents selected from the group consisting of halo, cyano, R^{x} and OR^{x};
   (b) C₃₋₆ cycloalkyl, which is optionally substituted with one to four substituents selected from the group consisting of halo and R^{x};
   (c) heteroaryl, which can be monocyclic or bicyclic, which is optionally substituted with one or two substituents selected from the group consisting of halo and R^{x}; and
   (d) heterocyclyl, which can be monocyclic or bicyclic;
R⁶ is selected from the group consisting of hydrogen, halo, hydroxy and C₁₋₆ alkyl;
R⁷ is selected from the group consisting of hydrogen, halo, hydroxy and C₁₋₆ alkyl;
R^{x} is hydrogen or C₁₋₆ alkyl, which is optionally substituted with one to three substituents selected from the group consisting of halo and hydroxy;
or a pharmaceutically acceptable salt thereof.

In an embodiment of the invention, Q is -CH₂-. In another embodiment of the invention, Q is absent.

The present invention also relates to compounds of Formula Ia: 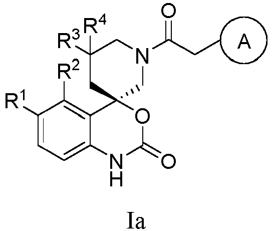 wherein 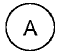 is 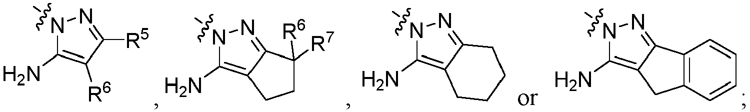
R¹ is selected from the group consisting of hydrogen, halo, hydroxy and C₁₋₆ alkyl;
R² is selected from the group consisting of hydrogen, halo, hydroxy and C₁₋₆ alkyl;
R³ is selected from the group consisting of hydrogen, halo, hydroxy and C₁₋₆ alkyl;
R⁴ is selected from the group consisting of hydrogen, halo, hydroxy and C₁₋₆ alkyl;
R⁵ is selected from the group consisting of
   (a) phenyl, which is optionally substituted with one to three substituents selected from the group consisting of halo, cyano, R^{x} and OR^{x};
   (b) C₃₋₆ cycloalkyl, which is optionally substituted with one to four substituents selected from the group consisting of halo and R^{x};
   (c) heteroaryl, which can be monocyclic or bicyclic, which is optionally substituted with one or two substituents selected from the group consisting of halo and R^{x}; and
   (d) heterocyclyl, which can be monocyclic or bicyclic;
R⁶ is selected from the group consisting of hydrogen, halo, hydroxy and C₁₋₆ alkyl;
R⁷ is selected from the group consisting of hydrogen, halo, hydroxy and C₁₋₆ alkyl;
R^{x} is hydrogen or C₁₋₆ alkyl, which is optionally substituted with one to three substituents selected from the group consisting of halo and hydroxy;
or a pharmaceutically acceptable salt thereof.

The present inventnion also relates to compounds of Formula 1b: 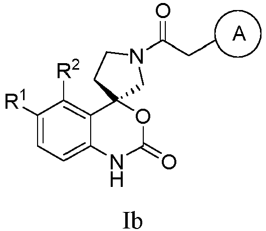 wherein 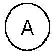 is 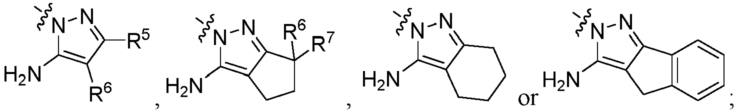
R¹ is selected from the group consisting of hydrogen, halo, hydroxy and C₁₋₆ alkyl;
R² is selected from the group consisting of hydrogen, halo, hydroxy and C₁₋₆ alkyl;
R⁵ is selected from the group consisting of
   (a) phenyl, which is optionally substituted with one to three substituents selected from the group consisting of halo, cyano, R^{x} and OR^{x};
   (b) C₃₋₆ cycloalkyl, which is optionally substituted with one to four substituents selected from the group consisting of halo and R^{x};
   (c) heteroaryl, which can be monocyclic or bicyclic, which is optionally substituted with one or two substituents selected from the group consisting of halo and R^{x}; and
   (d) heterocyclyl, which can be monocyclic or bicyclic;
R⁶ is selected from the group consisting of hydrogen, halo, hydroxy and C₁₋₆ alkyl;
R⁷ is selected from the group consisting of hydrogen, halo, hydroxy and C₁₋₆ alkyl;
R^{x} is hydrogen or C₁₋₆ alkyl, which is optionally substituted with one to three substituents selected from the group consisting of halo and hydroxy,
or a pharmaceutically acceptable salt thereof

In an embodiment of the invention, 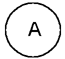 is 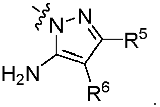 In another embodiment of the invention, 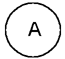 is 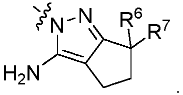 In another embodiment of the invention, 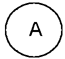 is 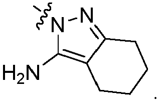 In another embodiment of the invention, 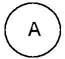 is

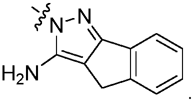

In an embodiment of the invention, R¹ is halo. In a class of the embodiment, R¹ is chloro. In another class of the embodiment, R¹ is fluoro.

In an embodiment of the invention, R² is halo. In a class of the embodiment, R² is chloro. In another class of the embodiment, R² is fluoro.

In an embodiment of the invention, R³ is hydrogen.

In an embodiment of the invention, R⁴ is hydrogen.

In an embodiment of the invention, R⁵ is selected from the group consisting of
(a) phenyl, which is optionally substituted with one to three substituents selected from the group consisting of halo, cyano, methyl, methoxy, difluoromethoxy, trifluoromethoxy and trifluoromethyl;
(b) cyclopropyl, cyclobutyl, cyclopentyl or cyclohexyl, wherein said cyclopropyl is optionally substituted with one to four substituents selected from the group consisting of methyl and halo;
(c) benzodioxyl, pyridinyl, pyridazinyl, pyrimidinyl, thiophenyl, furanyl, thiazolyl, pyrrolyl, benzofuranyl, pyrazolyl or isoxazolyl, wherein said pyridinyl, pyrazolyl, pyrrolyl and isoxazolyl groups are optionally substituted with one or two substituents selected from the group consisting of halo, methyl and trifluoromethyl; and
(d) tetrahydropyranyl or oxabicyclohexanyl.

In a class of the invention, R⁵ is phenyl, which is optionally substituted with one to three substituents selected from the group consisting of halo, cyano, methyl, methoxy, difluoromethoxy, trifluoromethoxy and trifluoromethyl.

In an embodiment of the invention, R⁶ is hydrogen. In another embodiment of the invention, R⁶ is halo. In another embodiment of the invention, R⁶ is methyl.

In an embodiment of the invention, R⁷ is hydrogen. In another embodiment of the invention, R⁷ is methyl.

Reference to the preferred classes and subclasses set forth above is meant to include all combinations of particular and preferred groups unless stated otherwise.

Specific embodiments of the present invention include, but are not limited to the compounds identified herein as Examples 1 to 67 or pharmaceutically acceptable salts thereof.

Also included within the scope of the present invention is a pharmaceutical composition which is comprised of a compound of Formula I, Ia or Ib as described above and a pharmaceutically acceptable carrier. The invention is also contemplated to encompass a pharmaceutical composition which is comprised of a pharmaceutically acceptable carrier and any of the compounds specifically disclosed in the present application. These and other aspects of the invention will be apparent from the teachings contained herein.

The invention includes compositions for treating diseases or condition in which plasma kallikrein activity is implicated. Accordingly the invention includes compositions for treating impaired visual activity, diabetic retinopathy, wet age-related macular degeneration, diabetic macular edema, retinal vein occlusion, hereditary angioedema, diabetes, pancreatitis, cerebral hemorrhage, nephropathy, cardiomyopathy, neuropathy, inflammatory bowel disease, arthritis, inflammation, septic shock, hypotension, cancer, adult respiratory distress syndrome, disseminated intravascular coagulation, blood coagulation during cardiopulmonary bypass surgery, and bleeding from postoperative surgery in a mammal, comprising a compound of the invention in a pharmaceutically acceptable carrier. A class of the invention includes compositions for treating hereditary angioedema, uveitis, posterior uveitis, wet age-related macular degeneration, diabetic macular edema, diabetic retinopathy and retinal vein occlusion. These compositions may optionally include anti-inflammatory agents, anti-VEGF agents, immunosuppressive agents, anticoagulants, antiplatelet agents, and thrombolytic agents. The compositions can be added to blood, blood products, or mammalian organs in order to effect the desired inhibitions.

The invention also includes compositions for preventing or treating retinal vascular permeability associated with diabetic retinopathy and diabetic macular edema in a mammal, comprising a compound of the invention in a pharmaceutically acceptable carrier. These compositions may optionally include anti-inflammatory agents, anti-VEGF agents, immunosuppressive agents, anticoagulants, antiplatelet agents, and thrombolytic agents.

The invention also includes compositions for treating inflammatory conditions of the eye, which includes, but is not limited to, uveitis, posterior uveitis, macular edema, acute macular degeneration, wet age-related macular degeneration, retinal detachments, retinal vein occlusion, ocular tumors, fungal infections, viral infections, multifocal choroiditis, diabetic uveitis, diabetic macular edema, diabetic retinopathy, proliferative vitreoretinopathy, sympathetic opthalmia, Vogt Koyanagi-Harada syndrome, histoplasmosis and uveal diffusion. These compositions may optionally include anti-inflammatory agents, anti-VEGF agents, immunosuppressive agents, anticoagulants, antiplatelet agents, and thrombolytic agents.

The invention also includes compositions treating posterior eye disease, which includes, but is not limited to, uveitis, posterior uveitis, wet age-related macular degeneration, diabetic macular edema, diabetic retinopathy and retinal vein occlusion. These compositions may optionally include anti-inflammatory agents, anti-VEGF agents, immunosuppressive agents, anticoagulants, antiplatelet agents, and thrombolytic agents.

It will be understood that the invention is directed to the compounds of structural Formula I, Ia and Ib described herein, as well as the pharmaceutically acceptable salts of the compounds of structural Formula I, Ia and Ib and also salts that are not pharmaceutically acceptable when they are used as precursors to the free compounds or their pharmaceutically acceptable salts or in other synthetic manipulations.

The compounds of the present invention may be administered in the form of a pharmaceutically acceptable salt. The term "pharmaceutically acceptable salt" refers to salts prepared from pharmaceutically acceptable non-toxic bases or acids including inorganic or organic bases and inorganic or organic acids. Salts of basic compounds encompassed within the term "pharmaceutically acceptable salt" refer to non-toxic salts of the compounds of this invention which are generally prepared by reacting the free base with a suitable organic or inorganic acid. Representative salts of basic compounds of the present invention include, but are not limited to, the following: acetate, ascorbate, adipate, alginate, aspirate, benzenesulfonate, benzoate, bicarbonate, bisulfate, bitartrate, borate, bromide, butyrate, camphorate, camphorsulfonate, camsylate, carbonate, chloride, clavulanate, citrate, cyclopentane propionate, diethylacetic, digluconate, dihydrochloride, dodecylsulfanate, edetate, edisylate, estolate, esylate, ethanesulfonate, formic, fumarate, gluceptate, glucoheptanoate, gluconate, glutamate, glycerophosphate, glycollylarsanilate, hemisulfate, heptanoate, hexanoate, hexylresorcinate, hydrabamine, hydrobromide, hydrochloride, 2-hydroxyethanesulfonate, hydroxynaphthoate, iodide, isonicotinic, isothionate, lactate, lactobionate, laurate, malate, maleate, mandelate, mesylate, methylbromide, methylnitrate, methylsulfate, methanesulfonate, mucate, 2-naphthalene sulfonate, napsylate, nicotinate, nitrate, N-methylglucamine ammonium salt, oleate, oxalate, pamoate (embonate), palmitate, pantothenate, pectinate, persulfate, phosphate/diphosphate, pimelic, phenylpropionic, polygalacturonate, propionate, salicylate, stearate, sulfate, subacetate, succinate, tannate, tartrate, teoclate, thiocyanate, tosylate, triethiodide, trifluoroacetate, undeconate, valerate and the like. Furthermore, where the compounds of the invention carry an acidic moiety, suitable pharmaceutically acceptable salts thereof include, but are not limited to, salts derived from inorganic bases including aluminum, ammonium, calcium, copper, ferric, ferrous, lithium, magnesium, manganic, mangamous, potassium, sodium, zinc, and the like. Salts derived from pharmaceutically acceptable organic non-toxic bases include salts of primary, secondary, and *terti*ary amines, cyclic amines, dicyclohexyl amines and basic ion-exchange resins, such as arginine, betaine, caffeine, choline, N,N-dibenzylethylenediamine, diethylamine, 2-diethylaminoethanol, 2-dimethylaminoethanol, ethanolamine, ethylamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. Also, included are the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

These salts can be obtained by known methods, for example, by mixing a compound of the present invention with an equivalent amount and a solution containing a desired acid, base, or the like, and then collecting the desired salt by filtering the salt or distilling off the solvent. The compounds of the present invention and salts thereof may form solvates with a solvent such as water, ethanol, or glycerol. The compounds of the present invention may form an acid addition salt and a salt with a base at the same time according to the type of substituent of the side chain.

If the compounds of Formula I, Ia or Ib simultaneously contain acidic and basic groups in the molecule the invention also includes, in addition to the salt forms mentioned, inner salts or betaines (zwitterions).

The present invention encompasses all stereoisomeric forms of the compounds of Formula I, Ia and Ib. Unless a specific stereochemistry is indicated, the present invention is meant to comprehend all such stereoisomeric forms of these compounds. Centers of asymmetry that are present in the compounds of Formula I, Ia and Ib can all independently of one another have (R) configuration or (S) configuration. When bonds to the chiral carbon are depicted as straight lines in the structural Formulas of the invention, it is understood that both the (R) and (S) configurations of the chiral carbon, and hence both each individual enantiomer and mixtures thereof, are embraced within the Formula. When a particular configuration is depicted, that entantiomer (either (R) or (S), at that center) is intended. Similarly, when a compound name is recited without a chiral designation for a chiral carbon, it is understood that both the (R) and (S) configurations of the chiral carbon, and hence individual enantiomers and mixtures thereof, are embraced by the name. The production of specific stereoisomers or mixtures thereof may be identified in the Examples where such stereoisomers or mixtures were obtained, but this in no way limits the inclusion of all stereoisomers and mixtures thereof from being within the scope of this invention.

Unless a specific enantionmer or diastereomer is indicated, the invention includes all possible enantiomers and diastereomers and mixtures of two or more stereoisomers, for example mixtures of enantiomers and/or diastereomers, in all ratios. Thus, enantiomers are a subject of the invention in enantiomerically pure form, both as levorotatory and as dextrorotatory antipodes, in the form of racemates and in the form of mixtures of the two enantiomers in all ratios. In the case of a cis/trans isomerism the invention includes both the cis form and the trans form as well as mixtures of these forms in all ratios. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at an intermediate step during the synthesis of a compound of Formula I, Ia or Ib, or it can be done on a final racemic product. Absolute stereochemistry may be determined by X-ray crystallography of crystalline products or crystalline intermediates which are derivatized, if necessary, with a reagent containing a stereogenic center of known configuration. Where compounds of this invention are capable of tautomerization, all individual tautomers as well as mixtures thereof are included in the scope of this invention. The present invention includes all such stereoisomers, as well as salts, solvates (including hydrates) and solvated salts of such racemates, enantiomers, diastereomers and tautomers and mixtures thereof.

In the compounds of the invention, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the specifically and generically described compounds. For example, different isotopic forms of hydrogen (H) include protium (1_{H}) and deuterium (2_{H}). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds can be prepared without undue experimentation by conventional techniques well known to those skilled in the art or by processes analogous to those described in the general process schemes and examples herein using appropriate isotopically-enriched reagents and/or intermediates.

When any variable (e.g. R^{x}, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is bicyclic, it is intended that the bond be attached to any of the suitable atoms on either ring of the bicyclic moiety.

It is understood that one or more silicon (Si) atoms can be incorporated into the compounds of the instant invention in place of one or more carbon atoms by one of ordinary skill in the art to provide compounds that are chemically stable and that can be readily synthesized by techniques known in the art from readily available starting materials. Carbon and silicon differ in their covalent radius leading to differences in bond distance and the steric arrangement when comparing analogous C-element and Si-element bonds. These differences lead to subtle changes in the size and shape of silicon-containing compounds when compared to carbon. One of ordinary skill in the art would understand that size and shape differences can lead to subtle or dramatic changes in potency, solubility, lack of off-target activity, packaging properties, and so on. (Diass, J. O. et al. Organometallics (2006) 5:1188-1198; Showell, G.A. et al. Bioorganic & Medicinal Chemistry Letters (2006) 16:2555-2558).

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" (with one or more substituents) should be understood as meaning that the group in question is either unsubstituted or may be substituted with one or more substituents.

Furthermore, compounds of the present invention may exist in amorphous form and/or one or more crystalline forms, and as such all amorphous and crystalline forms and mixtures thereof of the compounds of Formula I, Ia and Ib are intended to be included within the scope of the present invention. In addition, some of the compounds of the instant invention may form solvates with water (i.e., a hydrate) or common organic solvents. Such solvates and hydrates, particularly the pharmaceutically acceptable solvates and hydrates, of the instant compounds are likewise encompassed within the scope of this invention, along with un-solvated and anhydrous forms.

Accordingly, the compounds within the generic structural formulas, embodiments and specific compounds described and claimed herein encompass salts, all possible stereoisomers and tautomers, physical forms (e.g., amorphous and crystalline forms), solvate and hydrate forms thereof and any combination of these forms.

Except where noted herein, the terms "alkyl" and "alkylene" are intended to include both branched- and straight-chain saturated aliphatic hydrocarbon groups having the specified number of carbon atoms. Commonly used abbreviations for alkyl groups are used throughout the specification, e.g. methyl, may be represented by conventional abbreviations including "Me" or CH₃ or a symbol that is an extended bond as the terminal group, e.g. " 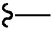 " , ethyl may be represented by "Et" or CH₂CH₃, propyl may be represented by "Pr" or CH₂CH₂CH₃, butyl may be represented by "Bu" or CH₂CH₂CH₂CH₃, etc. "C₁₋₄ alkyl" (or "C₁-C₄ alkyl") for example, means linear or branched chain alkyl groups, including all isomers, having the specified number of carbon atoms. For example, the structures 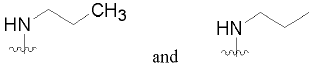 have equivalent meanings. C₁₋₄ alkyl includes n-, iso-, sec- and t-butyl, n- and isopropyl, ethyl and methyl. If no number is specified, 1-4 carbon atoms are intended for linear or branched alkyl groups.

Except where noted, the term "cycloalkyl" means a monocyclic or bicyclic saturated aliphatic hydrocarbon group having the specified number of carbon atoms. For example, "cycloalkyl" includes cyclopropyl, cyclobutyl, cyclopentyl, cyclohexyl, and so on.

Except where noted, the term "heteroaryl", as used herein, represents a stable monocyclic or bicyclic ring system of up to 10 atoms in each ring, wherein at least one ring is aromatic, and at least one ring contains from 1 to 4 heteroatoms selected from the group consisting of O, N and S. Bicyclic heteroaryl ring systems include fused ring systems, where two rings share two atoms, and spiro ring systems, where two rings share one atom. Heteroaryl groups within the scope of this definition include but are not limited to: azaindolyl, benzoimidazolyl, benzisoxazolyl, benzofuranyl, benzofurazanyl, benzopyrazolyl, benzotriazolyl, benzothiophenyl, benzoxazolyl, carbazolyl, carbolinyl, cinnolinyl, dihydroindenyl, furanyl, indolinyl, indolyl, indolazinyl, indazolyl, isobenzofuranyl, isoindolyl, isoquinolyl, isothiazolyl, isoxazolyl, naphthalenyl, naphthpyridinyl, oxadiazolyl, oxazolyl, oxazoline, isoxazoline, pyranyl, pyrazinyl, pyrazolyl, pyrazolopyrimidinyl, pyridazinyl, pyridopyridinyl, pyridinyl, pyridyl, pyrimidinyl, pyrrolyl, quinazolinyl, quinolyl, quinoxalinyl, tetrahydronaphthyridinyl, tetrazolyl, tetrazolopyridyl, thiadiazolyl, thiazolyl, thienyl, triazolyl, dihydrobenzoimidazolyl, dihydrobenzofuranyl, dihydrobenzothiophenyl, dihydrobenzoxazolyl, dihydroindolyl, dihydroquinolinyl, dihydrobenzodioxinyl, dihydropyrazoloxazinyl, dihydropyrazolyothiazinedioxidyl, methylenedioxybenzene, benzothiazolyl, benzothienyl, quinolinyl, isoquinolinyl, oxazolyl, tetra-hydroquinoline, sulfolanyl, 1,3-benzodioxolyl, 3-oxo-3,4dihydro-2N-benzo[b][1,4]thiazine, imidazopyridinyl, 2-oxo-2,3-dihydroimidazolyl, 3,4-dihydrobenzoxazinyl, 2-oxo-2,3-dihydrooxazolyl, dihydroisobenzofuranyl, 1-oxoisoindolinyl, dioxido-2,3-dihydrobenzoisothiazolyl, 2-oxopyridyl, tetrahydroisoquinolyl, tetrahydrothiazolo[5,4-c]pyridyl, 4-oxo-dihydroquinazolinyl, dihydro-1'H-spirocyclopropane-1,4'-isoquinolyl. If the heteroaryl contains nitrogen atoms, it is understood that the corresponding N-oxides thereof are also encompassed by this definition.

The term "heterocycle" or "heterocyclyl" as used herein is intended to mean a stable nonaromatic monocyclic or bicyclic ring system of up to 10 atoms in each ring, unless otherwise specified, containing from 1 to 4 heteroatoms selected from the group consisting of O, N, S, SO, or SO₂. Bicyclic heterocyclic ring systems include fused ring systems, where two rings share two atoms, and spiro ring systems, where two rings share one atom. "Heterocyclyl" therefore includes, but is not limited to the following: azabicyclohexanyl, azaspirohexanyl, azaspirononanyl, azaspirooctanyl, azetidinyl, dioxanyl, isochromanyl, oxadiazaspirodecenyl, oxaspirooctanyl, oxazolidinonyl, 2-oxo-azabicyclo[3.1.0]hexanyl, piperazinyl, piperidinyl, pyrrolidinyl, morpholinyl, thiomorpholinyl, tetrahydrofurnayl, tetrahydropyranyl, dihydropiperidinyl, tetrahydrothiophenyl and the like. If the heterocycle contains a nitrogen, it is understood that the corresponding N-oxides thereof are also encompassed by this definition.

Except where noted, the term "halogen" or "halo" means fluorine, chlorine, bromine or iodine.

"Celite^{®}" (Fluka) diatomite is diatomaceous earth, and can be referred to as "celite".

Except where noted herein, structures containing substituent variables such as variable "R" below: 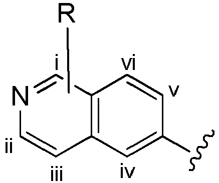 which are depicted as not being attached to any one particular bicyclic ring carbon atom, represent structures in which the variable can be optionally attached to any bicyclic ring carbon atom. For example, variable R shown in the above structure can be attached to any one of 6 bicyclic ring carbon atoms i, ii, iii, iv, v or vi.

Except where noted herein, bicyclic ring systems include fused ring systems, where two rings share two atoms, and spiro ring systems, where two rings share one atom.

The invention also relates to medicaments containing at least one compound of the Formula I, Ia and Ib and/or of a pharmaceutically acceptable salt of the compound of the Formula I, Ia and Ib and/or an optionally stereoisomeric form of the compound of the Formula I, Ia and Ib or a pharmaceutically acceptable salt of the stereoisomeric form of the compound of Formula I, Ia and Ib, together with a pharmaceutically suitable and pharmaceutically acceptable vehicle, additive and/or other active substances and auxiliaries.

The term "patient" used herein is taken to mean mammals such as primates, humans, sheep, horses, cattle, pigs, dogs, cats, rats, and mice.

The medicaments according to the invention can be administered by oral, inhalative, rectal or transdermal administration or by subcutaneous, intraarticular, intraperitoneal or intravenous injection. Oral administration is preferred. Coating of stents with compounds of the Formulas I and other surfaces which come into contact with blood in the body is possible.

The invention also relates to a process for the production of a medicament, which comprises bringing at least one compound of the Formula I or Ia into a suitable administration form using a pharmaceutically suitable and pharmaceutically acceptable carrier and optionally further suitable active substances, additives or auxiliaries.

Suitable solid or galenical preparation forms are, for example, granules, powders, coated tablets, tablets, (micro)capsules, suppositories, syrups, juices, suspensions, emulsions, drops or injectable solutions and preparations having prolonged release of active substance, in whose preparation customary excipients such as vehicles, disintegrants, binders, coating agents, swelling agents, glidants or lubricants, flavorings, sweeteners and solubilizers are used. Frequently used auxiliaries which may be mentioned are magnesium carbonate, titanium dioxide, lactose, mannitol and other sugars, talc, lactose, gelatin, starch, cellulose and its derivatives, animal and plant oils such as cod liver oil, sunflower, peanut or sesame oil, polyethylene glycol and solvents such as, for example, sterile water and mono- or polyhydric alcohols such as glycerol.

The dosage regimen utilizing the plasma kallikrein inhibitors is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Oral dosages of the plasma kallikrein inhibitors, when used for the indicated effects, will range between about 0.01 mg per kg of body weight per day (mg/kg/day) to about 30 mg/kg/day, preferably 0.025-7.5 mg/kg/day, more preferably 0.1-2.5 mg/kg/day, and most preferably 0.1-0.5 mg/kg/day (unless specificed otherwise, amounts of active ingredients are on free base basis). For example, an 80 kg patient would receive between about 0.8 mg/day and 2.4 g/day, preferably 2-600 mg/day, more preferably 8-200 mg/day, and most preferably 8-40 mg/ day. A suitably prepared medicament for once a day administration would thus contain between 0.8 mg and 2.4 g, preferably between 2 mg and 600 mg, more preferably between 8 mg and 200 mg, and most preferably 8 mg and 40 mg, e.g., 8 mg, 10 mg, 20 mg and 40 mg. Advantageously, the plasma kallikrein inhibitors may be administered in divided doses of two, three, or four times daily. For administration twice a day, a suitably prepared medicament would contain between 0.4 mg and 4 g, preferably between 1 mg and 300 mg, more preferably between 4 mg and 100 mg, and most preferably 4 mg and 20 mg, e.g., 4 mg, 5 mg, 10 mg and 20 mg.

Intravenously, the patient would receive the active ingredient in quantities sufficient to deliver between 0.025-7.5 mg/kg/day, preferably 0.1-2.5 mg/kg/day, and more preferably 0.1-0.5 mg/kg/day. Such quantities may be administered in a number of suitable ways, e.g. large volumes of low concentrations of active ingredient during one extended period of time or several times a day, low volumes of high concentrations of active ingredient during a short period of time, e.g. once a day. Typically, a conventional intravenous formulation may be prepared which contains a concentration of active ingredient of between about 0.01-1.0 mg/mL, e.g. 0.1 mg/mL, 0.3 mg/mL, and 0.6 mg/mL, and administered in amounts per day of between 0.01 mL/kg patient weight and 10.0 mL/kg patient weight, e.g. 0.1 mL/kg, 0.2 mL/kg, 0.5 mL/kg. In one example, an 80 kg patient, receiving 8 mL twice a day of an intravenous formulation having a concentration of active ingredient of 0.5 mg/mL, receives 8 mg of active ingredient per day. Glucuronic acid, L-lactic acid, acetic acid, citric acid or any pharmaceutically acceptable acid/conjugate base with reasonable buffering capacity in the pH range acceptable for intravenous administration may be used as buffers. The choice of appropriate buffer and pH of a formulation, depending on solubility of the drug to be administered, is readily made by a person having ordinary skill in the art.

Compounds of Formula I, Ia and Ib can be administered both as a monotherapy and in combination with other therapeutic agents, including but not limited to anti-inflammatory agents, anti-VEGF agents, immunosuppressive agents, anticoagulants, antiplatelet agents, and thrombolytic agents.

An "anti-inflammatory agent" is any agent which is directly or indirectly effective in the reduction of inflammation when administered at a therapeutically effective level. "Anti-inflammatory agent" includes, but is not limited to steroidal anti-inflammatory agents and glucocorticoids. Suitable anti-inflammatory agents include, but are not limited to, cortisone, dexamethasone, hydrocortisone, methylprednisolone, prednisolone, prednisone and triamcinolone.

An "anti-VEGF agent" is any agent which is directly or indirectly effective in inhibiting the activity of VEGF (Vascular Endothelial Growth Factor). Suitable anti-VEGF agents include, but are not limited to, bevacizumab, ranibizumab, brolucizumab and aflibercept.

An "immunosuppressant agent" is any agent which is directly or indirectly effective in suppressing, or reducing, the strength of the body's immune system. Suitable immunosuppressant agents include, but are not limited to, corticosteroids (for example, prednisone, budesonide, prednisolone), janus kinase inhibitors (for example, tofacitinib), calcineurin inhibitors (for example, cyclosporin, tacrolimus), mTOR inhibitors (for example, sirolimus, everolimus), IMDH inhibitors (for example, azathioprine, leflunomide, mycophenolate), biologics (for example, abatacept, adalimumab, anakinra, certolizumab, etanercept, golimumab, infliximab, ixekizumab, natalizumab, rituximab, secukinumab, tocilizumab, ustekinumab, vedolizumab), and monoclonal antibodies (for example, basiliximab, daclizumab).

Suitable anticoagulants include, but are not limited to, factor XIa inhibitors, thrombin inhibitors, thrombin receptor antagonists, factor VIIa inhibitors, factor Xa inhibitors, factor IXa inhibitors, factor XIIa inhibitors, adenosine diphosphate antiplatelet agents (e.g., P2Y12 antagonists), fibrinogen receptor antagonists (e.g. to treat or prevent unstable angina or to prevent reocclusion after angioplasty and restenosis), other anticoagulants such as aspirin, and thrombolytic agents such as plasminogen activators or streptokinase to achieve synergistic effects in the treatment of various vascular pathologies. Such anticoagulants include, for example, apixaban, dabigatran, cangrelor, ticagrelor, vorapaxar, clopidogrel, edoxaban, mipomersen, prasugrel, rivaroxaban, and semuloparin. For example, patients suffering from coronary artery disease, and patients subjected to angioplasty procedures, would benefit from coadministration of fibrinogen receptor antagonists and thrombin inhibitors.

In certain embodiments the anti-inflammatory agents, anti-VEGF agents, immunosuppressant agents, anticoagulants, antiplatelet agents, and thrombolytic agents described herein are employed in their conventional dosage ranges and regimens as reported in the art, including, for example, the dosages described in editions of the Physicians' Desk Reference, such as the 70th edition (2016) and earlier editions. In other embodiments, the anti-inflammatory agents, anti-VEGF agents, immunosuppressant agents, anticoagulants, antiplatelet agents, and thrombolytic agents described herein are employed in lower than their conventional dosage ranges.

Alternatively or additionally, one or more additional pharmacologically active agents may be administered in combination with a compound of the invention. The additional active agent (or agents) is intended to mean a pharmaceutically active agent (or agents) that is active in the body, including pro-drugs that convert to pharmaceutically active form after administration, which is different from the compound of the invention, and also includes free-acid, free-base and pharmaceutically acceptable salts of said additional active agents when such forms are sold commercially or are otherwise chemically possible. Generally, any suitable additional active agent or agents, including but not limited to anti-hypertensive agents, additional diuretics, anti-atherosclerotic agents such as a lipid modifying compound, anti-diabetic agents and/or anti-obesity agents may be used in any combination with the compound of the invention in a single dosage formulation (a fixed dose drug combination), or may be administered to the patient in one or more separate dosage formulations which allows for concurrent or sequential administration of the active agents (co-administration of the separate active agents). Examples of additional active agents which may be employed include but are not limited to angiotensin converting enzyme inhibitors (e.g, alacepril, benazepril, captopril, ceronapril, cilazapril, delapril, enalapril, enalaprilat, fosinopril, imidapril, lisinopril, moveltipril, perindopril, quinapril, ramipril, spirapril, temocapril, or trandolapril); angiotensin II receptor antagonists also known as angiotensin receptor blockers or ARBs, which may be in free-base, free-acid, salt or pro-drug form, such as azilsartan, e.g., azilsartan medoxomil potassium (EDARBI^{®}), candesartan, e.g., candesartan cilexetil (ATACAND^{®}), eprosartan, e.g., eprosartan mesylate (TEVETAN^{®}), irbesartan (AVAPRO^{®}), losartan, e.g., losartan potassium (COZAAR^{®}), olmesartan, e.g, olmesartan medoximil (BENICAR^{®}), telmisartan (MICARDIS^{®}), valsartan (DIOVAN^{®}), and any of these drugs used in combination with a thiazide-like diuretic such as hydrochlorothiazide (e.g., HYZAAR^{®}, DIOVAN HCT^{®}, ATACAND HCT^{®}), etc.); potassium sparing diuretics such as amiloride HCl, spironolactone, epleranone, triamterene, each with or without HCTZ; neutral endopeptidase inhibitors (e.g., thiorphan and phosphoramidon); aldosterone antagonists; aldosterone synthase inhibitors; renin inhibitors; enalkrein; RO 42-5892; A 65317; CP 80794; ES 1005; ES 8891; SQ 34017; aliskiren (2(S),4(S),5(S),7(S)-N-(2-carbamoyl-2-methylpropyl)-5-amino-4-hydroxy-2,7-diisopropyl-8-[4-methoxy-3-(3-methoxypropoxy)-phenyl]-octanamid hemifumarate) SPP600, SPP630 and SPP635); endothelin receptor antagonists; vasodilators (e.g. nitroprusside); calcium channel blockers (e.g., amlodipine, nifedipine, verapamil, diltiazem, felodipine, gallopamil, niludipine, nimodipine, nicardipine); potassium channel activators (e.g., nicorandil, pinacidil, cromakalim, minoxidil, aprilkalim, loprazolam); sympatholitics; beta-adrenergic blocking drugs (e.g., acebutolol, atenolol, betaxolol, bisoprolol, carvedilol, metoprolol, metoprolol tartate, nadolol, propranolol, sotalol, timolol); alpha adrenergic blocking drugs (e.g., doxazosin, prazosin or alpha methyldopa); central alpha adrenergic agonists; peripheral vasodilators (e.g. hydralazine); lipid lowering agents, e.g., HMG-CoA reductase inhibitors such as simvastatin and lovastatin which are marketed as ZOCOR^{®} and MEVACOR^{®} in lactone pro-drug form and function as inhibitors after administration, and pharmaceutically acceptable salts of dihydroxy open ring acid HMG-CoA reductase inhibitors such as atorvastatin (particularly the calcium salt sold in LIPITOR^{®}), rosuvastatin (particularly the calcium salt sold in CRESTOR^{®}), pravastatin (particularly the sodium salt sold in PRAVACHOL^{®}), and fluvastatin (particularly the sodium salt sold in LESCOL^{®}); a cholesterol absorption inhibitor such as ezetimibe (ZETIA^{®}), and ezetimibe in combination with any other lipid lowering agents such as the HMG-CoA reductase inhibitors noted above and particularly with simvastatin (VYTORIN^{®}) or with atorvastatin calcium; niacin in immediate-release or controlled release forms, and particularly niacin in combination with a DP antagonist such as laropiprant and/or with an HMG-CoA reductase inhibitor; niacin receptor agonists such as acipimox and acifran, as well as niacin receptor partial agonists; metabolic altering agents including insulin sensitizing agents and related compounds for the treatment of diabetes such as biguanides (e.g., metformin), meglitinides (e.g., repaglinide, nateglinide), sulfonylureas (e.g., chlorpropamide, glimepiride, glipizide, glyburide, tolazamide, tolbutamide), thiazolidinediones also referred to as glitazones (e.g., pioglitazone, rosiglitazone), alpha glucosidase inhibitors (e.g., acarbose, miglitol), dipeptidyl peptidase inhibitors, (e.g., sitagliptin (JANUVIA^{®}), alogliptin, vildagliptin, saxagliptin, linagliptin, dutogliptin, gemigliptin), ergot alkaloids (e.g., bromocriptine), combination medications such as JANUMET^{®} (sitagliptin with metformin), and injectable diabetes medications such as exenatide and pramlintide acetate; inhibitors of glucose uptake, such as sodium-glucose transporter (SGLT) inhibitors and its various isoforms, such as SGLT-1, SGLT-2 (e.g., ASP-1941, TS-071, BI-10773, tofogliflozin, LX-4211, canagliflozin, dapagliflozin, ertugliflozin, ipragliflozin, remogliflozin and sotagliflozin), and SGLT-3; or with other drugs beneficial for the prevention or the treatment of the above-mentioned diseases including but not limited to diazoxide; and including the free-acid, free-base, and pharmaceutically acceptable salt forms, pro-drug forms, e.g., esters, and salts of pro-drugs of the above medicinal agents, where chemically possible. Trademark names of pharmaceutical drugs noted above are provided for exemplification of the marketed form of the active agent(s); such pharmaceutical drugs could be used in a separate dosage form for concurrent or sequential administration with a compound of the invention, or the active agent(s) therein could be used in a fixed dose drug combination including a compound of the invention.

Typical doses of the plasma kallikrein inhibitors of the invention in combination with other suitable agents may be the same as those doses of plasma kallikrein inhibitors administered without coadministration of additional agents, or may be substantially less that those doses of plasma kallikrein inhibitors administered without coadministration of additional agents, depending on a patient's therapeutic needs.

The compounds are administered to a mammal in a therapeutically effective amount. By "therapeutically effective amount" it is meant an amount of a compound of the present invention that, when administered alone or in combination with an additional therapeutic agent to a mammal, is effective to treat (i.e., prevent, inhibit or ameliorate) the disease condition or treat the progression of the disease in a host.

The compounds of the invention are preferably administered alone to a mammal in a therapeutically effective amount. However, the compounds of the invention can also be administered in combination with an additional therapeutic agent, as defined below, to a mammal in a therapeutically effective amount. When administered in a combination, the combination of compounds is preferably, but not necessarily, a synergistic combination. Synergy, as described for example by Chou and Talalay, Adv. Enzyme Regul. 1984, 22, 27-55, occurs when the effect (in this case, inhibition of the desired target) of the compounds when administered in combination is greater than the additive effect of each of the compounds when administered individually as a single agent. In general, a synergistic effect is most clearly demonstrated at suboptimal concentrations of the compounds. Synergy can be in terms of lower cytotoxicity, increased anticoagulant effect, or some other beneficial effect of the combination compared with the individual components.

By "administered in combination" or "combination therapy" it is meant that the compound of the present invention and one or more additional therapeutic agents are administered concurrently to the mammal being treated. When administered in combination each component may be administered at the same time or sequentially in any order at different points in time. Thus, each component may be administered separately but sufficiently closely in time so as to provide the desired therapeutic effect. The administration of each component does not need to be via the same route of administration; for example, one component can be administered orally, and another can be delivered into the vitreous of the eye.

### GENERAL METHODS

Compounds of the present invention may be prepared using conventional techniques or according to the methodology outlined in the following general synthetic schemes. One skilled in the art can vary the procedures and reagents shown to arrive at similar intermediates and/or final compounds.

NMR spectra were measured on VARIAN or Bruker NMR Systems (400, 500 or 600 MHz). Chemical shifts are reported in ppm downfield and up field from tetramethylsilane (TMS) and referenced to either internal TMS or solvent resonances (¹H NMR: δ 7.27 for C*D*Cl₃, δ 2.50 for (CD₃)(C*H*D₂)SO, and ¹³C NMR: δ 77.02 for *C*DCl₃, δ 39.51 for (*C*D₃)₂SO. Coupling constants (*J*) are expressed in hertz (Hz), and spin multiplicities are given as s (singlet), d (doublet), dd (double doublet), t (triplet), m (multiplet), and br (broad). Chiral resolutions can be performed on either Waters Thar 80 SFC or Berger MG II preparative SFC systems. LC-MS data can be recorded on SHIMADAZU LC-MS-2020, SHIMADAZU LC-MS-2010, or Agilent 1100 series LC-MS, Agilent Prime-1260, or Waters Acquity LC-MS instruments using C18 columns employing a MeCN gradient in water containing 0.02 to 0.1% TFA. UV detections were at 220 and/or 254 nm and ESI ionization was used for MS detection.

When chiral resolution was achieved by chromatography using chiral columns, the chiral columns used for SFC chiral resolutions are listed in tables. Some of the chiral columns used were CHIRALPAK AD, CHIRALCEL OJ, CHIRALPAK AS, CHIRALPAK AY, CHIRALPAK IA, CHIRALPAK AD-H, and CHIRALPAK AS-H. Henceforth, they will be referred by their two or three letter abbreviations. As a convention, the fast-eluting isomer from a chiral resolution is always listed first in this table followed immediately by the slower-eluting isomer from the same resolution. If more than two isomers were separated, they will be always listed in the tables in order they were eluted, such as Peak 1 followed by Peak 2, Peak 3 and so on. A * symbol near a chiral center in a structure denotes that this chiral center was resolved by chiral resolution without its stereochemical configuration unambiguously determined.

Reagents used in the following procedures include: Selectfluor^{™} which is (1-chloromethyl-4-fluoro-1,4-diazoniabicyclo [2.2.2]octane bis (tetrafluoroborate), and is available from Sigma Aldrich.

Also, TLC is thin layer chromatography; UV is ultraviolet; W is watts; wt. % is percentage by weight; x g is times gravity; α_{D} is the specific rotation of polarized light at 589 nm; °C is degrees Celsius; % w/v is percentage in weight of the former agent relative to the volume of the latter agent; cpm is counts per minute; δ_{H} is chemical shift; Rochelle's salt is potassium sodium tartrate; MS is mass spectrum, and a mass spectrum obtained by ES-MS may be denoted herein by "LC-MS"; *m*/*z* is mass to charge ratio.

For purposes of this specification, the following abbreviations have the indicated meanings:
- Ac: Acetyl
- ACN: Acetonitrile
- aq: Aqueous
- Boc or BOC: tert-butoxycarbonyl
- br: Broad
- Bu or ⁿBu: Butyl (normal)
- °C: degrees Celsius
- calcd.: Calculated
- δ: chemical shift
- d: Doublet
- DBU: 1,8-diazabicyclo[5.4.0]undec-7-ene
- DCM: Dichloromethane
- dd: doublet of doublets
- ddd: doublet of a doublet of doublets
- ddt: doublet of a doublet of triplets
- DIEA or Hünig's base: *N*,*N*-diisopropylethylamine
- DMAP: 4-dimethylaminopyridine
- DMF: Dimethylformamide
- DMSO: dimethyl sulfoxide
- dt: doublet of triplets
- DTT: Dithiothreitol
- EDTA: ethylenediamine tetraacetic acid
- eq: Equivalents
- ESI: electrospray ionization
- Et: ethyl
- EtOH: ethanol
- EtOAc: ethyl acetate
- g: grams
- GST: glutathione S-transferase
- h: hour
- HATU: *N,N,N',N'*-tetramethyl-*O*-(7-azabenzotriazol-1-yl)uronium hexafluorophosphate
- HPLC: high-performance liquid chromatography
- Hz: Hertz
- *J*: coupling constant
- KHMDS: potassium hexamethyldisilazide
- LC: liquid chromatography
- LCMS: liquid chromatography mass spectrometry
- m: multiplet
- M: molar
- Me: methyl
- MeOH: methanol
- mg: milligrams
- MHz: megahertz
- min: minute
- µL: microliters
- mL: milliliters
- mM: millimolar
- mmol: millimoles
- MS: mass spectrometry
- *N*: nitrogen substituted
- NCS: N-chlorosuccinimide
- nm: nanometer
- NMR: nuclear magnetic resonance spectroscopy
- OAc: acetate
- pH: measure of acidity or basicity of aq. or other solutions
- Ph: phenyl
- Pr: propyl
- q: quartet
- *rac*: racemic mixture
- RT or rt: room temperature (ambient, about 25 °C)
- s: singlet
- satd.: saturated
- SFC: supercritical fluid chromatography
- t: triplet
- *^{t}*Bu: *tert*-butyl
- td: triplet of doublets
- *tert*: tertiary
- TFA: trifluoroacetic acid
- THF: tetrahydrofuran
- TLC: thin layer chromatography
- Tris: tris(hydroxymethyl)aminomethane

### General

Starting materials used were obtained from commercial sources or prepared in other examples, unless otherwise noted.

The methods used for the preparation of the compounds of this invention are illustrated by the following schemes. Unless specified otherwise, all starting materials used are commercially available.

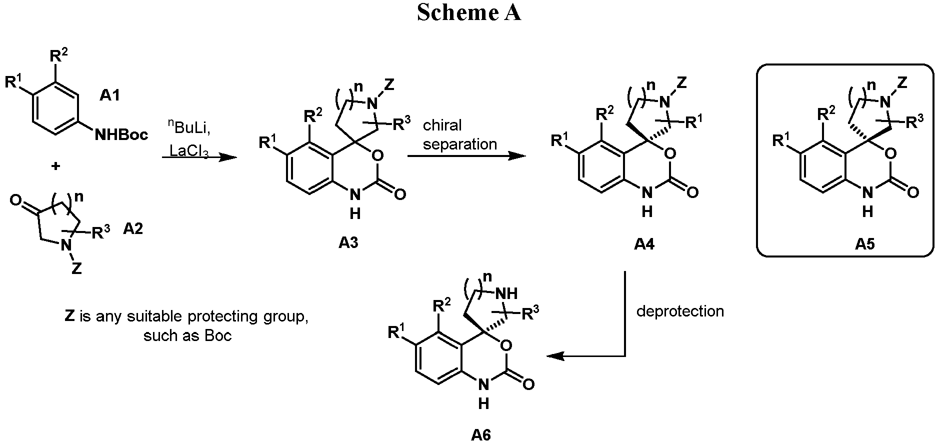

**Scheme A** illustrates the synthetic sequence for preparation of substituted spirocarbamates such as **A6** from Boc-protected aniline **A1** and ketones such as **A2.** Directed lithiation of aniline **A1** and addition into the heterocyclic ketone **A2** occurs in the presence of a Lewis acid (LaCl₃, e.g.). The tertiary alcohol undergoes in situ cyclization onto the carbamate to give spirocarbamate derivatives such as **A3,** which can be subjected to chiral separation, preferably using supercritical flow chromatography (SFC) to afford enantiomers **A4** and **A5.** Deprotection of either enantiomer **(A4,** e.g.) gives the secondary amine **A6.**

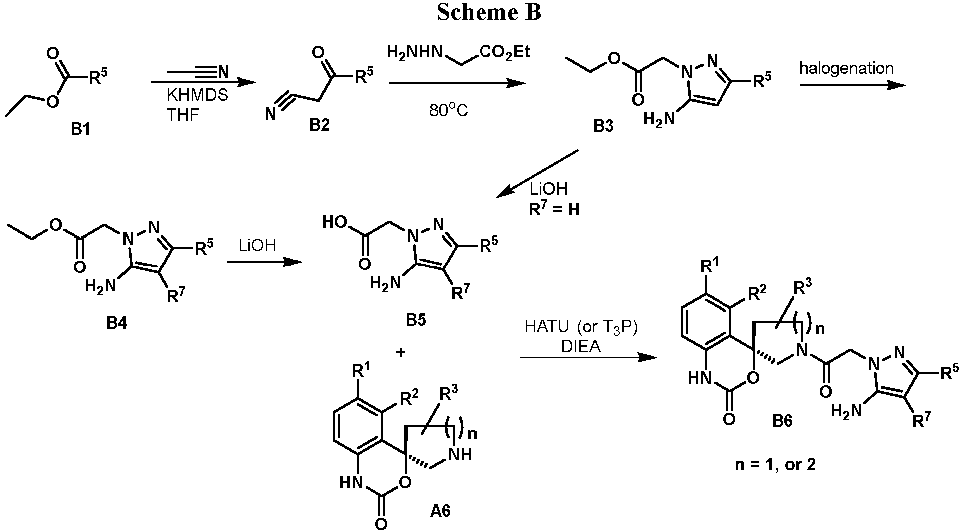

**Scheme B** illustrates the synthetic sequence for preparation of substituted aminopyrazole pyrrolidine (or piperidine) spirocarbamates such as **B6** from amino pyrazole acetic acid derivatives **B5** and spirocarbamates such as **A6.** The amino pyrazole intermediates **B5** are prepared starting from heating β-nitrile ketones **B2** with ethyl aminoglycinate, followed by halogenation and hydrolysis of the ester. Non-commercially available **B2** are prepared from the corresponding esters such as **B1**.

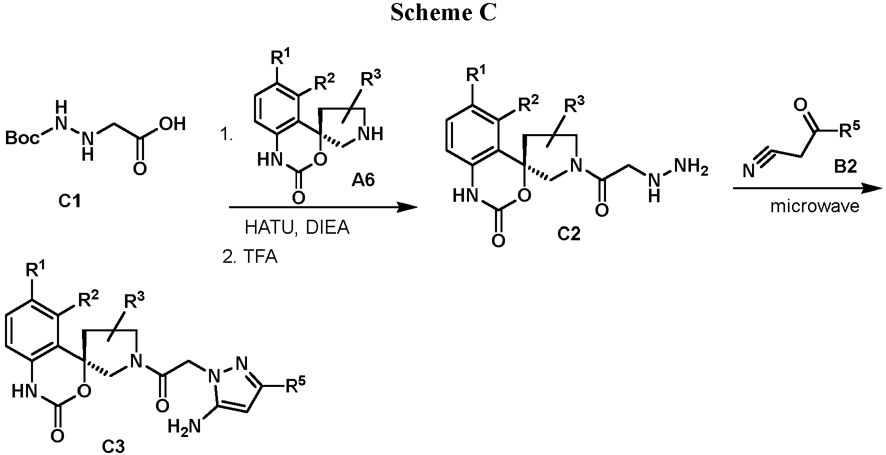

**Scheme C** illustrates the alternative synthetic route for preparation of substituted aminopyrazole pyrrolidine spirocarbamates such as **C3** by mixing the hydrazine key intermediate **C2** and β-nitrile ketones **B2** under microwave irradiation. Hydrazine key intermediate **C2** is prepared from the amide coupling of commercially available ((*tert*-butoxycarbonyl)amino)glycine C1 and substituted pyrrolidine (or piperidine) spirocarbamates such as **A6** with HATU and DIEA, followed by de-Boc protection with TFA.

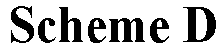

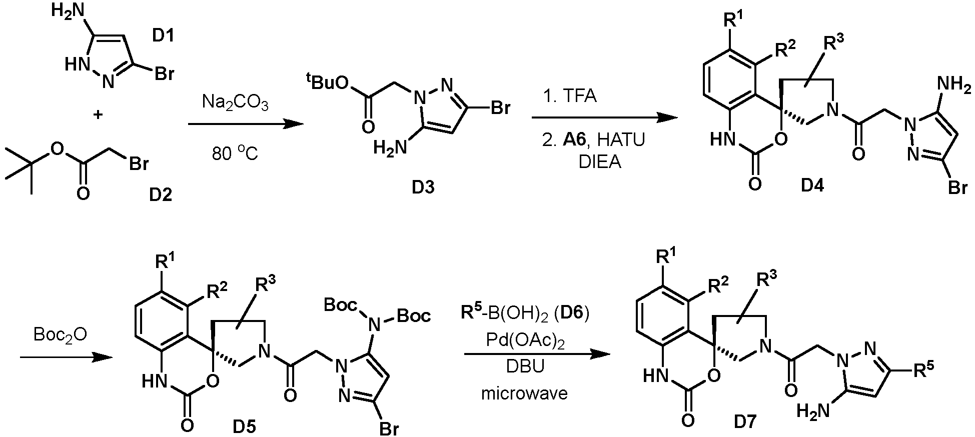

**Scheme D** illustrates the alternative synthetic route for preparation of substituted aminopyrazole pyrrolidine spirocarbamates such as **D7** from the key bromopyrazole intermediate **D5** and aryl boronic acids **(D6)** by a coupling reaction under microwave irradiation. The precursor **D5** is prepared starting from the alkylation of the commercially available bromo substituted aminopyrazole **D1** with the bromoacetate **D2.** The obtained product **D3** is then hydrolyzed to the corresponding acid and coupled with the spirocarbamates such as **A6.**

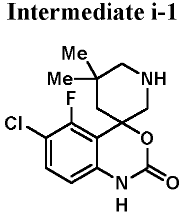

### 6-Chloro-5-fluoro-5',5'-dimethylspiro[benzo[d][1,3]oxazine-4,3'-piperidin]-2(1H)-one

Step 1: *tert*-Butyl 6-chloro-5-fluoro-5',5'-dimethyl-2-oxo-1,2-dihydrospiro[benzo[*d*][1,3]oxazine-4,3'-piperidine]-1'-carboxylate: THF (55 mL) was added to *tert*-butyl (4-chloro-3-fluorophenyl)carbamate (2.21 g, 9.0 mmol) under a N₂ atmosphere. The solution was cooled to -78 °C. ⁿBuLi (11 mL, 2.5 M in hexanes, 27.9 mmol) was added over 40 min. The reaction mixture was allowed to stir at -78 °C for an additional 45 min, then, a solution of LaCl₃•2LiCl (22.5 mL, 0.6 M in THF, 13.5 mmol) and *tert*-butyl 3,3-dimethyl-5-oxopiperidine-1-carboxylate (3.1 g, 13.5 mmol) was added over a period of 40 min to the reaction mixture at -78 °C. The reaction mixture was allowed to warm to rt and stirred for 16 h. KO*^{t}*Bu (5.3 mL, 1.7 M in THF, 9.0 mmol) was added to the reaction mixture, the solution heated to 60 °C and then stirred for an additional 3 h. The flask was cooled to rt, quenched with 1 M HCl and diluted with EtOAc. The layers were separated and the aq. phase extracted with EtOAc. The combined organic layers were dried over MgSO₄, filtered and concentrated under reduced pressure. The crude residue was purified by column chromatography (silica gel, hexanes:EtOAc) to afford the title compound. LCMS [M+Na]⁺ = 421.1 (Calcd. 421.1).

### Step 2: 6-Chloro-5-fluoro-5',5'-dimethylspiro[benzo[d][1,3]oxazine-4,3'-piperidin]-2(1H)-one:

HCl (25 mL, 4 M in dioxane, 100 mmol) was added to a suspension of *tert*-butyl 6-chloro-5-fluoro-5',5'-dimethyl-2-oxo-1,2-dihydrospiro[benzo[*d*][1,3]oxazine-4,3'-piperidine]-1'-carboxylate (7.98 g, 20 mmol) in 1,4-dioxane (30 mL). The reaction mixture was heated to 90 °C and stirred for 12 h. On complete deprotection by LCMS, the flask was cooled to rt, and the solvent removed under reduced pressure to give the crude title compound. The crude product was carried forward to the next step without further purification. LCMS [M+H]⁺ = 299.1 (Calcd. 299.1).

The following compounds were prepared using procedures similar to those described for **Intermediate i-1** using the appropriate starting materials.

| **Intermediate** | **Structure** | **Name** | **LCMS [M+H]⁺** | **Chiral Column for Boc-protected precursor** |
|---|---|---|---|---|
| **i-1a** | 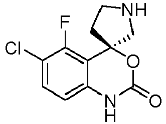 | (*R*)-6-chloro-5-fluorospiro[benzo[*d*][1,3]oxazine-4,3'-pyrrolidin]-2(1*H*)-one | Calcd. 257.0, found 257.0 | AD-H; peak B |
| **i-1b** | 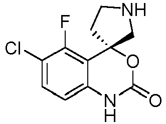 | (*S*)-6-Chloro-5-fluorospiro[benzo[*d*][1,3]oxazine-4,3'-pyrrolidin]-2(1*H*)-one | Calcd. 257.0, found 257.0 | AD-H; peak A |
| **i-1c** | 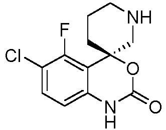 | (*R*)-6-Chloro-5-fluorospiro[benzo[*d*][1,3]oxazine-4,3'-piperidin]-2(1*H*)-one | Calcd. 271.1, found 271.0 | AD-H; peak B |
| **i-1d** | 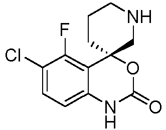 | (S)-6-Chloro-5-fluorospiro[benzo[*d*][1,3]oxazine-4,3'-piperidin]-2(1*H*)-one | Calcd. 271.1, found 271.0 | AD-H, peak A |

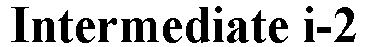

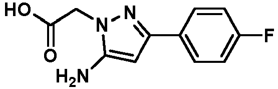

### 2-(5-Amino-3-(4-fluorophenyl)-1H-pyrazol-1-yl)acetic acid

Step 1: Ethyl 2-(5-amino-3-(4-fluorophenyl)-1*H*-pyrazol-1-yl)acetate: A mixture of ethyl aminoglycinate hydrochloride (155 mg, 1.00 mmol) and 3-(4-fluorophenyl)-3-oxopropanenitrile (163 mg, 1.00 mmol) in EtOH (1.3 mL) was heated to reflux for 2 h. The reaction mixture was cooled to rt, and concentrated to dryness under vacuum. The resulting residue was then partitioned between EtOAc and satd. aq. NaHCO₃. The aq. layer was extracted with EtOAc, and the combined organic layers were dried over Na₂SO₄, and concentrated to afford the title compound. The crude product was carried on without purification. LCMS [M+H]⁺ = 264.1 (Calcd. 264.1)

Step 2: 2-(5-Amino-3-(4-fluorophenyl)-1*H*-pyrazol-1-yl)acetic acid: To a solution of ethyl 2-(5-amino-3-(4-fluorophenyl)-1*H*-pyrazol-1-yl)acetate (245 mg, 0.930 mmol) in 1,4-dioxane (2.7 mL), 1M aq. NaOH (4.6 mL, 4.6 mmol) was added, and the resulting mixture was heated to 50 °C. After 30 min, the reaction was cooled to rt, acidified to pH 4 with 1M HCl, and extracted using EtOAc. The organic layer was passed through Na₂SO₄, and concentrated to dryness to afford the title compound, which was carried forward crude to the next step. LCMS [M+H]⁺= 236.1 (Calcd. 236.1)

The following compounds were prepared using procedures similar to those described for **Intermediate i-2** using the appropriate starting materials

| **Intermediate** | **Structure** | **Name** | **LCMS [M+H]⁺** |
|---|---|---|---|
| **i-2a** | 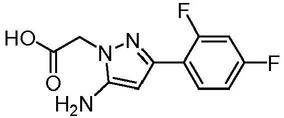 | 2-(5-Amino-3-(2,4-difluorophenyl)-1*H*-pyrazol-1-yl)acetic acid | Calcd. 254.1, found 254.1 |
| **i-2b** | 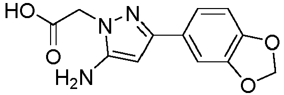 | 2-(5-Amino-3-(benzo[*d*][1,3]dioxol-5-yl)-1*H*-pyrazol-1-yl)acetic acid | Calcd.262.1, found 262.0 |
| **i-2c** | 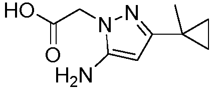 | 2-(5-Amino-3-(1-methylcyclopropyl)-1*H-*pyrazol-1-yl)acetic acid | Calcd. 196.1, found 196.1 |
| **i-2d** | 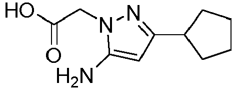 | 2-(5-Amino-3-cyclopentyl-1*H*-pyrazol-1-yl)acetic acid | Calcd. 210.1, found 210.1 |
| **i-2e** | 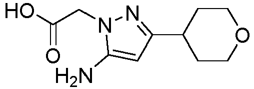 | 2-(5-Amino-3 -(tetrahydro-2*H*-pyran-4-yl)-1*H*-pyrazol-1-yl)acetic acid | Calcd. 226.1, found 226.1 |
| **i-2f** | 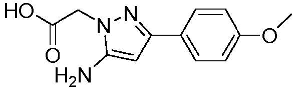 | 2-(5-Amino-3-(4-methoxyphenyl)-1*H*-pyrazol-1-yl)acetic acid | Calcd. 248.1, found 248.1 |
| **i-2g** | 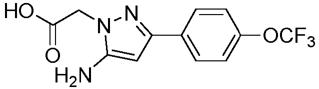 | 2-(5-Amino-3-(4-(trifluoromethoxy)phenyl)-1*H*-pyrazol-1-yl)acetic acid | Calcd. 302.0, found 302.1 |
| **i-2h** | 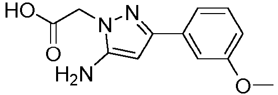 | 2-(5-Amino-3-(3-methoxyphenyl)-1*H*-pyrazol-1-yl)acetic acid | Calcd. 248.1, found 248.1 |
| **i-2i** | 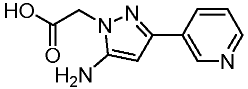 | 2-(5-Amino-3 -(pyridin-3-yl)-1*H*-pyrazol-1-yl)acetic acid | Calcd. 219.1, found 219.1 |
| **i-2j** | 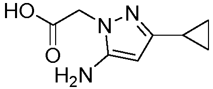 | 2-(5-Amino-3-cyclopropyl-1*H*-pyrazol-1-yl)acetic acid | Calcd. 182.0, found 182.1 |
| **i-2k** | 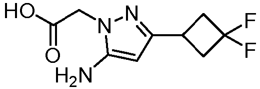 | 2-(5-Amino-3-(3,3-difluorocyclobutyl)-1*H-*pyrazol-1-yl)acetic acid | Calcd. 232.0, found 232.1 |
| **i-2l** | 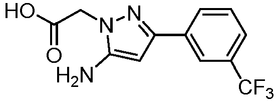 | 2-(5-Amino-3-(3-(trifluoromethyl)phenyl)-1*H-*pyrazol-1-yl)acetic acid | Calcd. 285.9, found 286.1 |
| **i-2m** | 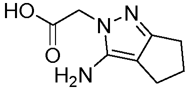 | 2-(3-Amino-5,6-dihydrocyclopenta[c]pyrazol-2(4*H*)-yl)acetic acid | Calcd. 182.0, found 182.1 |
| **i-2n** | 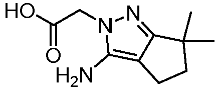 | 2-(3-Amino-6,6-dimethyl-5,6-dihydrocyclopenta[c]pyrazol-2(4*H*)-yl)acetic acid | Calcd. 210.1, found 210.1 |
| **i-2o** | 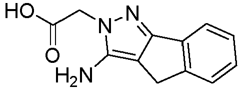 | 2-(3-Aminoindeno[1,2-c]pyrazol-2(4*H*)-yl)acetic acid | Calcd. 230.1, found 230.1 |
| | | | |
| **i-2p** | 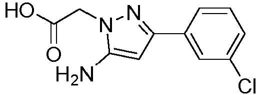 | 2-(5-Amino-3-(3-chlorophenyl)-1*H*-pyrazol-1-yl)acetic acid | Calcd. 252.1, found 252.1 |

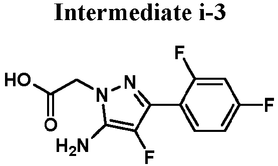

### 2-(5-Amino-3-(2,4-difluorophenyl)-4-fluoro-1H-pyrazol-1-yl)acetic acid

Step 1: Ethyl 2-(5-amino-3-(2,4-difluorophenyl)-1*H*-pyrazol-1-yl)acetate: A mixture of ethyl aminoglycinate hydrochloride (850 mg, 5.52 mmol) and 3-(2,4-difluorophenyl)-3-oxopropanenitrile (1.00 g, 5.52 mmol) in MeOH (12 mL) was heated in a microwave reactor at 100 °C for 20 min. The reaction mixture was cooled to rt, concentrated, and re-dissolved in EtOH (12 mL). An additional portion of ethyl aminoglycinate hydrochloride was added (0.15 eq, 116 mg), and the resulting mixture was heated under microwave irradiation at 100 °C for an additional 10 min. The reaction was cooled to rt and concentrated to afford a crude residue that was purified by column chromatography (silica, EtOAc/isohexane) to give the title compound. LCMS [M+H]⁺= 282.1 (Calcd. 282.1).

### Step 2: Ethyl 2-(5-amino-3-(2,4-difluorophenyl)-4-fluoro-1H-pyrazol-1-yl)acetate:

Selectfluor^{™} (846 mg, 2.38 mmol) and ethyl 2-(5-amino-3-(2,4-difluorophenyl)-1*H*-pyrazol-1-yl)acetate (672 mg, 2.38 mmol) were mixed in acetonitrile (24 mL) and stirred at rt overnight. The reaction was concentrated to afford a crude residue that was dissolved in EtOAc, washed with water and brine, dried (MgSO₄), filtered, and concentrated to afford a crude residue that was purified by column chromatography (silica, EtOAc/isohexane) to afford the title compound.
LCMS [M+H]⁺= 300.2 (Calcd. 300.1).waw

Step 3: 2-(5-Amino-3-(2,4-difluorophenyl)-4-fluoro-1*H*-pyrazol-1-yl)acetic acid: A solution of ethyl 2-(5-amino-3-(2,4-difluorophenyl)-4-fluoro-1*H*-pyrazol-1-yl)acetate (218 mg, 0.720 mmol) in a mixture of THF/MeOH/water (6 mL of a 1:4:1 mixture) was treated with LiOH (0.73 mL of a 2 M aq. solution, 1.46 mmol). The resulting mixture was allowed to stir at rt for 1 h, at which time, the reaction was concentrated to afford a crude residue that was redissolved in water, acidified with 1M HCl, and extracted with EtOAc. The organic layer was washed with brine, dried (MgSO₄), filtered and concentrated to obtain the title compound. LCMS [M+H]⁺= 272.1 (Calcd. 272.1).

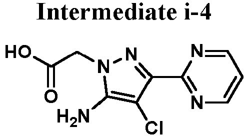

### 2-(5-Amino-4-chloro-3-(pyrimidin-2-yl)-1H-pyrazol-1-yl)acetic acid

Step 1: 3-Oxo-3-(pyrimidin-2-yl)propanenitrile: To a solution of ethyl pyrimidine-2-carboxylate (500 mg, 3.29 mmol) in THF (15 mL), acetonitrile (0.17 mL, 3.3 mmol) was added. The solution was cooled to -78 °C and KHMDS (3.3 mL of a 1.0 M THF solution, 3.3 mmol) was added dropwise. The resulting mixture was allowed to stir at -78 °C for 10 min, at which time the reaction was quenched with water, warmed to rt and neutralized with 1M HCl. The resulting mixture was extracted with EtOAc, and the organic layer was dried (MgSO₄) and concentrated to give the crude title compound that was carried on without purification. LCMS [M+H]⁺= 148.2 (Calcd. 148.0).

Step 2: Ethyl 2-(5-amino-3-(pyrimidin-2-yl)-1*H*-pyrazol-1-yl)acetate: To a solution of 3-Oxo-3-(pyrimidin-2-yl)propanenitrile 500 mg, 3.29 mmol) in EtOH (10 mL), ethyl aminoglycinate (388 mg, 3.29 mmol) was added, and the resulting mixture was heated at 100 °C for 1 h. The reaction mixture was cooled and concentrated to afford a crude residue that was purified by column chromatography (silica, ((1:3) EtOH:EtOAc):hexane) to yield the title compound. LCMS [M+H]⁺= 248.3 (Calcd. 248.1).

Step 3: Ethyl 2-(5-amino-4-chloro-3-(pyrimidin-2-yl)-1*H*-pyrazol-1-yl)acetate: To a solution of ethyl 2-(5-amino-3-(pyrimidin-2-yl)-1*H*-pyrazol-1-yl)acetate (200 mg, 0.809 mmol) in DMF (5 mL) at 0 °C, NCS (108 mg, 0.809 mmol) was added. The resulting mixture was stirred at 0 °C for 30 min. The reaction was diluted with EtOAc, and washed with satd. aq. NaHCO₃, followed by brine. The organic layer was dried (MgSO₄), and concentrated to afford a crude residue that was purified by column chromatography (silica, ((1:3) EtOH:EtOAc):hexane to yield the title compound. LCMS [M+H]⁺ = 282.3 (Calcd. 282.1).

Step 4: 2-(5-Amino-4-chloro-3-(pyrimidin-2-yl)-1*H*-pyrazol-1-yl)acetic acid: To a solution of ethyl 2-(5-amino-3-(pyrimidin-2-yl)-1*H*-pyrazol-1-yl)acetate (149 mg, 0.603 mmol) in THF (2 mL) at rt, LiOH (600 µL, 2M aq. solution 1.21 mmol) was added, and the resulting mixture was heated to 60 °C. After 1 h, the reaction was neutralized with 1M HCl and concentrated to afford the title compound which was carried on without further purification. LCMS [M+H]⁺ = 254.3 (Calcd. 254.0).

The following compounds were prepared using procedures similar to those described for **Intermediate i-4** using the appropriate starting materials

| **Intermediate** | **Structure** | **Name** | **LCMS [M+H]⁺** |
|---|---|---|---|
| **i-4a** | 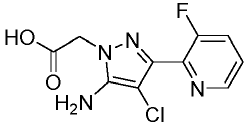 | 2-(5-Amino-4-chloro-3-(3-fluoropyridin-2-yl)-1*H-*pyrazol-1-yl)acetic acid | Calcd. 271.3, found 271.0 |
| **i-4b** | 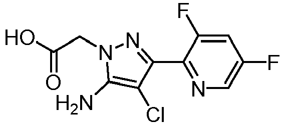 | 2-(5-Amino-4-chloro-3-(3,5-difluoropyridin-2-yl)-1*H*-pyrazol-1-yl)acetic acid | Calcd. 289.1, found 289.0 |
| **i-4c** | 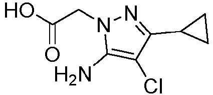 | 2-(5-Amino-4-chloro-3-cyclopropyl-1*H*-pyrazol-1-yl)acetic acid | Calcd. 216.1, found 216.0 |

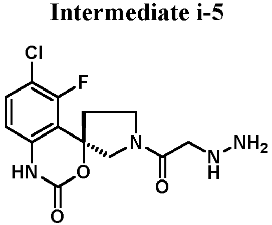

### (R)-1'-(Aminoglycyl)-6-chloro-5-fluorospiro[benzo[d][1,3]oxazine-4,3'-pyrrolidin]-2(1H)-one

Step 1: *tert*-Butyl (*R*)-2-(2-(6-chloro-5-fluoro-2-oxo-1,2-dihydrospiro[benzo[*d*][1,3]oxazine-4,3'-pyrrolidin]-1'-yl)-2-oxoethyl)hydrazine-1-carbonylate: A mixture of **i-1a** (2.37 g, 8.10 mmol) and DIEA (4.2 mL, 24.3 mmol) in DMF (20 mL) was added to a solution of HATU (3.69 g, 9.72 mmol) and ((*tert*-butoxycarbonyl)amino)glycine (1.54 g, 8.10 mmol) in DMF (40 mL). The resulting mixture was stirred at rt for 12 h, at which time the reaction was diluted with EtOAc, washed with water and brine, dried (MgSO₄), filtered, and concentrated. The crude residue was purified by column chromatography (silica, ((1:3)EtOH:EtOAc):hexanes to yield the title compound. LCMS [M+Na]⁺ = 451.2 (Calcd. 451.1).

Step 2: (*R*)-1 '-(Aminoglycyl)-6-chloro-5-fluorospiro[benzo[*d*][1,3]oxazine-4,3'-pyrrolidin]-2(1*H*)-one: TFA (3 mL) was added to a stirred solution of (*R*)-2-(2-(6-chloro-5-fluoro-2-oxo-1,2-dihydrospiro[benzo[*d*][1,3]oxazine-4,3'-pyrrolidin]-1'-yl)-2-oxoethyl)hydrazine-1-carboxylate (1.80 g, 3.92 mmol) in DCM (5 mL), and the resulting mixture was allowed to stir at rt. After 1 h, the reaction was concentrated to afford the title compound which was carried on without purification. LCMS [M+H]⁺ = 329.1 (Calcd. 329.1).

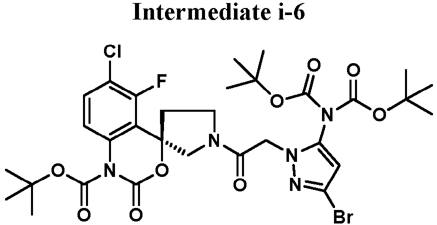

### (R)-1'-(2-(5-Amino-3-bromo-1H-pyrazol-1-yl)acetyl)-6-chloro-5-fluorospiro[benzo[d][1,3]oxazine-4,3'-pyrrolidin]-2(1H)-one (tris-Boc)

Step 1: *tert*-Butyl 2-(5-amino-3-bromo-1*H*-pyrazol-1-yl)acetate: To a mixture of Na₂CO₃ (7.46 g, 70.4 mmol) and 3-bromo-1*H*-pyrazol-5-amine (11.4 g, 70.4 mmol) in acetonitrile (150 mL) *tert-*butyl 2-bromoacetate (10.4 mL, 70.4 mmol) was added. The resulting mixture was heated to 80 °C for 60 h, at which time, the reaction was cooled to rt, filtered and concentrated. The crude residue was purified by column chromatography (silica, EtOAc:isohexane) to obtain title compound. LCMS [M+H]⁺ = 278.0 (Calcd. 278.0).

Step 2: (*R*)-1'-(2-(5-Amino-3-bromo-1*H*-pyrazol-1-yl)acetyl)-6-chloro-5-fluorospiro[benzo[*d*][1,3]oxazine-4,3'-pyrrolidin]-2(1*H*)-one: TFA (5.5 mL) was added to a stirred solution of *tert*-butyl 2-(5-amino-3-bromo-1*H*-pyrazol-1-yl)acetate (1.00 g, 3.62 mmol) in DCM (6 mL) at rt. After 3 h, the reaction was concentrated, and the resulting crude product was dissolved in DMF (25 mL). HATU (1.60 g, 4.35 mmol) and **i-1a** (1.06 g, 3.62 mmol) were added, followed by DIEA (2.5 mL, 14. 5mmol), and the reaction was stirred at rt for 12 h. The reaction mixture was diluted with EtOAc, washed with satd. aq. NaHCO₃ and brine, dried (MgSO₄), filtered, and concentrated to afford a crude residue that was purified by column chromatography (silica, EtOAc:isohexane) to yield the title compound. LCMS [M+H]⁺ = 459.9 (Calcd. 460.0).

Step 3: (*R*)-1'-(2-(5-Amino-3-bromo-1*H*-pyrazol-1-yl)acetyl)-6-chloro-5-fluorospiro[benzo[*d*][1,3]oxazine-4,3'-pyrrolidin]-2(1*H*)-one (*tris*-Boc): A mixture of (*R*)-1'-(2-(5-amino-3-bromo-1*H*-pyrazol-1-yl)acetyl)-6-chloro-5-fluorospiro[benzo[*d*][1,3]oxazine-4,3'-pyrrolidin]-2(1*H*)-one (1.00 g, 2.18 mmol), di-*tert*-butyl dicarbonate (4.4 mL, 8.7 mmol) and DMAP (0.133 g, 1.09 mmol) in DMF (15 ml) was stirred at rt. After 12 h, the reaction was diluted with EtOAc, washed with water and brine, dried (MgSO₄), filtered, and concentrated. The crude residue was purified by column chromatography (silica, EtOAc:isohexane) to yield the title compound. LCMS [M+Na]⁺ = 782.2 (Calcd. 782.2).

### Example 1

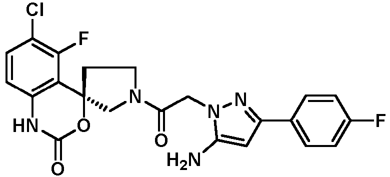

### (R)-1'-(2-(5-Amino-3-(4-fluorophenyl)-1H-pyrazol-1-yl)acetyl)-6-chloro-5-fluorospiro[benzo[d][1,3]oxazine-4,3'-pyrrolidin]-2(1H)-one 2,2,2-trifluoroacetate

1-Propanephosphonic anhydride (669 µL, 2.25 mmol) was added to a stirred solution of **i-1a** (528 mg, 1.80 mmol), **i-2** (353 mg, 1.50 mmol) and DIEA (0.80 mL, 4.5 mmol) in ACN (7.5 mL). The resulting mixture was allowed to stir at rt for 12 h, at which time, the reaction was concentrated and dissolved in EtOAc. The organics were washed successively with satd. aq. NH₄Cl, followed by satd. aq. NaHCO₃, and water. The organic layer was dried (Na₂SO₄), filtered and concentrated to afford a crude residue that was purified by preparative reverse phase HPLC (ACN/water + 0.1% TFA) to yield the title compound. ¹H NMR (600 MHz, CD₃CN) δ 8.56 - 8.48 (s, 1H), 7.81 - 7.73 (m, 2H), 7.48 - 7.40 (m, 1H), 7.22 - 7.15 (m, 2H), 6.77 (ddd, *J* = 17.4, 8.7, 1.3 Hz, 1H), 5.98 (d, *J =* 8.1 Hz, 1H), 5.04- 4.88 (m, 2H), 4.42 (br, 2H), 4.17 - 3.62 (m, 4H), 2.81 -2.50 (m, 2H). LCMS [M+H]⁺ = 474.0 (Calcd. 474.1).

The following compounds were prepared using procedures similar to those described in **Example 1** using the appropriate starting materials.

| **Example No.** | **Structure** | **Name** | **LCMS [M+H]⁺** |
|---|---|---|---|
| **2** | 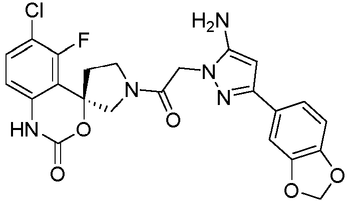 | (*R*)-1'-(2-(5-Amino-3-(benzo[*d*][1,3]dioxol-5-yl)-1*H*-pyrazol-1-yl)acetyl)-6-chloro-5-fluorospiro[benzo[*d*][1,3]ox azine-4,3'-pyrrolidin]-2(1*H*)-one | Calcd. 500.1, found 500.3 |
| **3** | 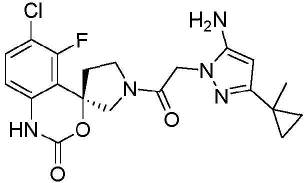 | (*R*)-1'-(2-(5-Amino-3-(1-methylcyclopropyl)-1*H-*pyrazol-1-yl)acetyl)-6-chloro-5-fluorospiro[benzo[*d*][1,3]ox azine-4,3'-pyrrolidin]-2(1*H*)-one | Calcd. 434.1, found 434.4 |
| **4** | 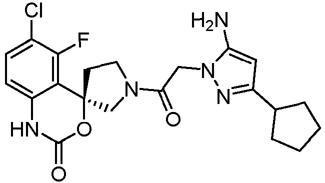 | (*R*)-1'-(2-(5-Amino-3-cyclopentyl-1H-pyrazol-1-yl)acetyl)-6-chloro-5-fluorospiro[benzo[d][1,3]ox azine-4,3'-pyrrolidin]-2(1H)-one | Calcd. 448.2, found 448.4 |
| **5** | 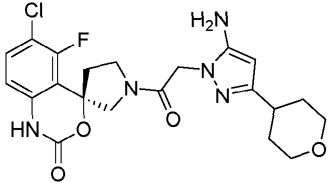 | (*R*)-1'-(2-(5-Amino-3-(tetrahydro-2*H*-pyran-4-yl)-1*H*-pyrazol-1-yl)acetyl)-6-chloro-5-fluorospiro[benzo[*d*][1,3]ox azine-4,3'-pyrrolidin]-2(1*H*)-one | Calcd. 464.1, found 464.4 |
| **6** | 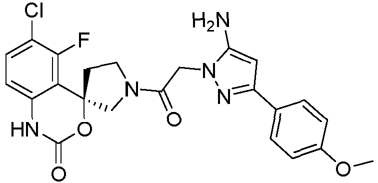 | (*R*)-1'-(2-(5-Amino-3-(4-methoxyphenyl)-1*H-*pyrazol-1-yl)acetyl)-6-chloro-5-fluorospiro[benzo[*d*][1,3]ox azine-4,3'-pyrrolidin]-2(1*H*)-one | Calcd. 486.1, found 486.3 |
| **7** | 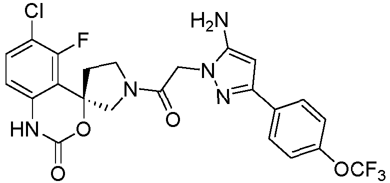 | (*R*)-1'-(2-(5-Amino-3-(4-(trifluoromethoxy)phenyl)-1*H*-pyrazol-1-yl)acetyl)-6-chloro-5-fluorospiro[benzo[*d*][1,3]ox azine-4,3'-pyrrolidin]-2(1*H*)-one | Calcd. 540.1, found 540.3 |
| **8** | 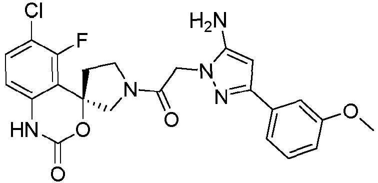 | (*R*)-1'-(2-(5-Amino-3-(3-methoxyphenyl)-1*H-*pyrazol-1-yl)acetyl)-6-chloro-5-fluorospiro[benzo[*d*][1,3]ox azine-4,3'-pyrrolidin]-2(1*H*)-one | Calcd. 486.1, found 486.3 |
| **9** | 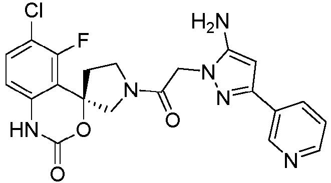 | (*R*)-1'-(2-(5-Amino-3-(pyridin-3-yl)-1*H*-pyrazol-1-yl)acetyl)-6-chloro-5-fluorospiro[benzo[*d*][1,3]ox azine-4,3'-pyrrolidin]-2(1*H*)-one | Calcd. 457.1, found 457.3 |
| **10** | 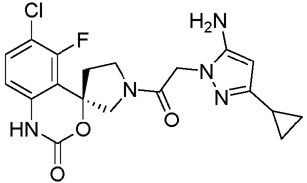 | (*R*)-1'-(2-(5-Amino-3-cyclopropyl-1*H*-pyrazol-1-yl)acetyl)-6-chloro-5-fluorospiro[benzo[*d*][3]oxaz ine-4,3'-]oxazine-4,3'-pyrrolidin]-2(1*H*)-one | Calcd. 420.1, found 420.4 |
| **11** | 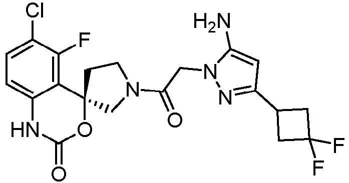 | (*R*)-1'-(2-(5-Amino-3-(3,3-difluorocyclobutyl)-1*H-*pyrazol-1-yl)acetyl)-6-chloro-5-fluorospiro[benzo[*d*][1,3]ox azine-4,3'-pyrrolidin]-2(1*H*)-one | Calcd. 470.1, found 470.4 |
| **12** | 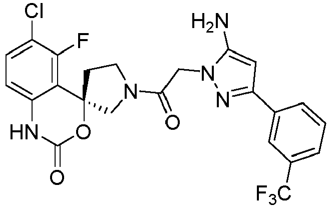 | (*R*)-1'-(2-(5-Amino-3-(3-(trifluoromethyl)phenyl)-1*H*-pyrazol-1-yl)acetyl)-6-chloro-5-fluorospiro[benzo[*d*][1,3]ox azine-4,3'-pyrrolidin]-2(1*H*)-one | Calcd. 524.1, found 524.3 |
| **13** | 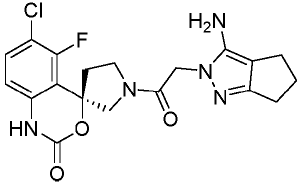 | (*R*)-1'-(2-(3-Amino-5,6-dihydrocyclopenta[*c*]pyrazo 1-2(4*H*)-yl)acetyl)-6-chloro-5-fluorospiro[benzo[*d*][1,3]ox azine-4,3'-pyrrolidin]-2(1*H*)-one | Calcd. 420.1, found 420.4 |
| **14** | 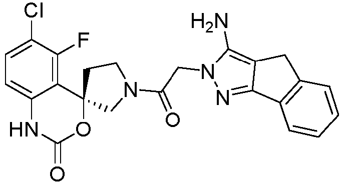 | (*R*)-1'-(2-(3-Aminoindeno[1,2-c]pyrazol-2(4*H*)-yl)acetyl)-6-chloro-5-fluorospiro[benzo[*d*][1,3]ox azine-4,3'-pyrrolidin]-2(1*H*)-one | Calcd. 468.1, found 468.4 |
| **15** | 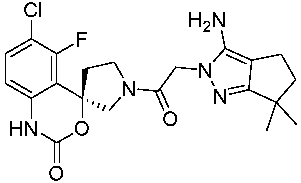 | (*R*)-1'-(2-(3-Amino-6,6-dimethyl-5,6-dihydrocyclopenta[*c*]pyrazo 1-2(4*H*)-yl)acetyl)-6-chloro-5-fluorospiro[benzo[*d*][1,3]ox azine-4,3'-pyrrolidin]-2(1*H*)-one | Calcd. 448.2, found 448.4 |
| **16** | 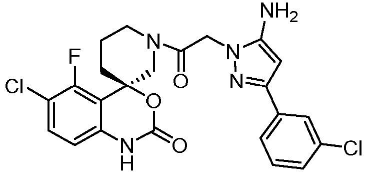 | (*R*)-1'-(2-(5-Amino-3-(3-chlorophenyl)-1*H*-pyrazol-1-yl)acetyl)-6-chloro-5-fluorospiro[benzo[*d*][1,3]ox azine-4,3'-piperidin]-2(1*H*)-one | Calcd. 504.1, found 504.3 |
| **17** | 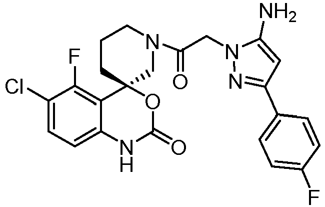 | (*R*)-1'-(2-(5-Amino-3-(4-fluorophenyl)-1*H*-pyrazol-1-yl)acetyl)-6-chloro-5-fluorospiro[benzo[*d*][1,3]ox azine-4,3'-piperidin]-2(1*H*)-one | Calcd. 488.1, found 488.4 |
| **18** | 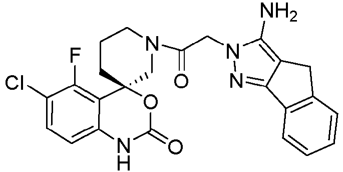 | (*R*)-1'-(2-(3-Aminoindeno[1,2-c]pyrazol-2(4*H*)-yl)acetyl)-6-chloro-5-fluorospiro[benzo[*d*][1,3]ox azine-4,3'-piperidin]-2(1*H*)-one | Calcd. 482.1, found 482.4 |

### Example 19

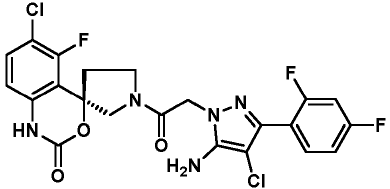

### (R)-1'-(2-(5-Amino-4-chloro-3-(2,4-difluorophenyl)-1H-pyrazol-1-yl)acetyl)-6-chloro-5-fluorospiro[benzo[d][1,3]oxazine-4,3'-pyrrolidin]-2(1H)-one 2,2,2-trifluoroacetate

1-Chloropyrrolidine-2,5-dione (13 mg, 0.099 mmol) was added to a solution of **i-2a** (25 mg, 0.099 mmol) in DMF (1 mL) at rt. After 45 min, HATU (45 mg, 0.12 mmol) was added, followed by **i-1a** (29 mg, 0.099 mmol) and DIEA (0.069 mL, 0.40 mmol), and the resulting mixture was allowed to stir at rt overnight. The reaction was purified by preparative reverse phase HPLC (ACN/water + 0.1% TFA) to obtain the title compound. ¹H NMR (500 MHz, DMSO-*d₆*) δ 10.80 - 10.76 (s, 1H), 7.55 (ddt, *J* = 29.2, 15.5, 8.5 Hz, 2H), 7.39 - 7.31 (m, 1H), 7.17 (td, *J* = 8.4, 2.5 Hz, 1H), 6.81 (dd, *J* = 10.8, 9.0 Hz, 1H), 5.01 4.83 (m, 2H), 4.18 - 4.02 (dd, *J =* 11.9 Hz, 1H), 3.96 - 3.92 (m, 1H), 3.83 - 3.51 (m, 4H), 2.65 - 2.47 (m, 2H). LCMS [M+H]⁺ = 526.2 (Calcd. 526.1).

The following compounds were prepared using procedures similar to those described in **Example 19** using the appropriate starting materials.

| **Example No.** | **Structure** | **Name** | **LCMS [M+H]⁺** |
|---|---|---|---|
| **20** | 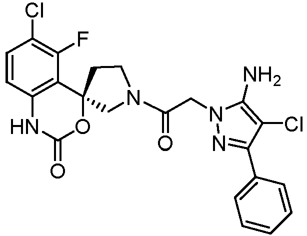 | (*R*)-1'-(2-(5-Amino-4-chloro-3-phenyl-1*H*-pyrazol-1-yl)acetyl)-6-chloro-5-fluorospiro[benzo[*d*][1,3]oxazine-4,3'-pyrrolidin]-2(1*H*)-one | Calcd. 490.1, found 489.9 |
| **21** | 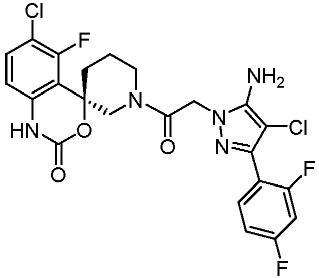 | (*R*)-1'-(2-(5-Amino-4-chloro-3-(2,4-difluorophenyl)-1*H*-pyrazol-1-yl)acetyl)-6-chloro-5-fluorospiro[benzo[*d*][1,3]oxazine-4,3'-piperidin]-2(1*H*)-one | Calcd. 540.1, found 540.2 |
| **22** | 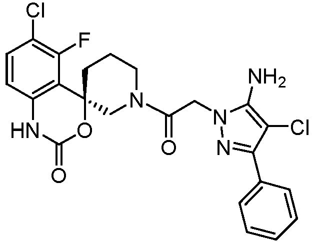 | (*R*)-1'-(2-(5-Amino-4-chloro-3-phenyl-1*H*-pyrazol-1-yl)acetyl)-6-chloro-5-fluorospiro[benzo[*d*][1,3]oxazine-4,3'-piperidin]-2(1*H*)-one | Calcd. 504.1, found 504.0 |

### Example 23

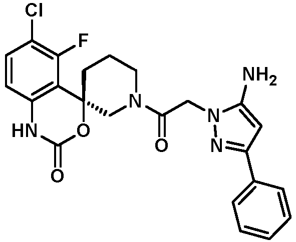

### (R)-1'-(2-(5-Amino-3-phenyl-1H-pyrazol-1-yl)acetyl)-6-chloro-5-fluorospiro[benzo[d][1,3]oxazine-4,3'-piperidin]-2(1H)-one

**i-1c** (25 mg, 0.081 mmol) was added to a mixture of 2-(5-amino-3-phenyl-1*H*-pyrazol-1-yl)acetic acid (18 mg, 0.081 mmol) and HATU (37 mg, 0.098 mmol) in DMF (0.6 mL), followed by DIEA (43 µL, 0.24 mmol). The mixture was stirred at rt for 5 h, at which time, the reaction was filtered and purified by preparative reverse phase HPLC (ACN/water + 0.1% TFA) to provide the title compound. ¹H NMR (500 MHz, DMSO-*d₆*) δ 10.81 - 10.66 (s, 1H), 7.74 - 7.68 (m, 2H), 7.57 (dt, *J* = 15.3, 8.2 Hz, 1H), 7.42 (t, *J* = 7.6 Hz, 2H), 7.34 (t, *J* = 7.3 Hz, 1H), 6.82 (dd, *J =* 13.7, 8.4 Hz, 1H), 5.90 (d, *J* = 10.5 Hz, 1H), 5.07 - 4.83 (m, 2H), 4.64 4.39 (m, 2H), 4.05 - 3.82 (m, 3H), 3.25 - 3.13 (td, *J =* 13.9 Hz, 1H), 2.77 -1.62 (m, 4H). LCMS [M+H]⁺ = 470.1 (Calcd. 470.1).

The following compounds were prepared using procedures similar to those described in **Example 23** using the appropriate starting materials.

| **Example No.** | **Structure** | **Name** | **LCMS [M+H]⁺** |
|---|---|---|---|
| **24** | 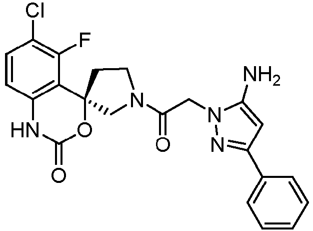 | (*R*)-1'-(2-(5-Amino-3-phenyl-1*H-*pyrazol-1-yl)acetyl)-6-chloro-5-fluorospiro[benzo[*d*][1,3]oxazine-4,3'-pyrrolidin]-2(1*H*)-one | Calcd. 456.1, found 456.0 |
| **25** | 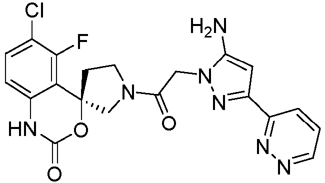 | (*R*)-1'-(2-(5-Amino-3-(pyridazin-3-yl)-1*H*-pyrazol-1-yl)acetyl)-6-chloro-5-fluorospiro[benzo[*d*][1,3]oxazine-4,3'-pyrrolidin]-2(1*H*)-one | Calcd. 458.1, found 458.1 |
| **26** | 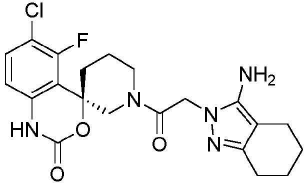 | (*R*)-1'-(2-(3-Amino-4,5,6,7-tetrahydro-2*H*-indazol-2-yl)acetyl)-6-chloro-5-fluorospiro[benzo[*d*][1,3]oxazine-4,3'-piperidin]-2(1*H*)-one | Calcd. 448.2, found 448.1 |
| **27** | 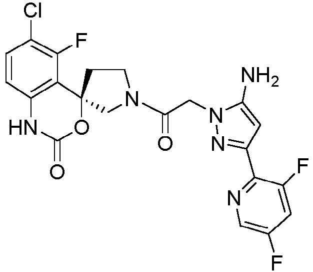 | (*R*)-1'-(2-(5-Amino-3-(3,5-difluoropyridin-2-yl)-1*H*-pyrazol-1-yl)acetyl)-6-chloro-5-fluorospiro[benzo[*d*][1,3]oxazine-4,3'-pyrrolidin]-2(1*H*)-one | Calcd. 493.1, found 493.0 |
| **28** | 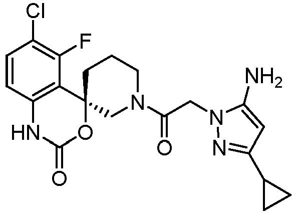 | (*R*)-1'-(2-(5-Amino-3-cyclopropyl-1*H*-pyrazol-1-yl)acetyl)-6-chloro-5-fluorospiro[benzo[*d*][1,3]oxazine-4,3'-piperidin]-2(1*H*)-one | Calcd. 434.1, found 434.1 |
| **29** | 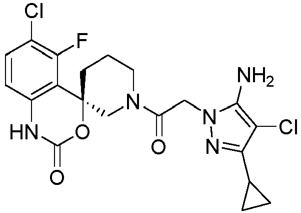 | (*R*)-1'-(2-(5-Amino-4-chloro-3-cyclopropyl-1*H*-pyrazol-1-yl)acetyl)-6-chloro-5-fluorospiro[benzo[*d*][1,3]oxazine-4,3'-piperidin]-2(1*H*)-one | Calcd. 468.1, found 468.0 |
| **30** | 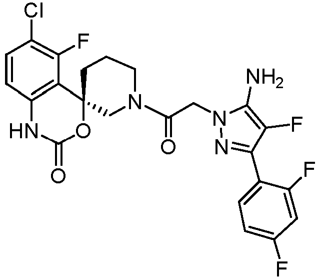 | (*R*)-1'-(2-(5-Amino-3-(2,4-difluorophenyl)-4-fluoro-1*H-*pyrazol-1-yl)acetyl)-6-chloro-5-fluorospiro[benzo[*d*][1,3]oxazine-4,3'-piperidin]-2(1*H*)-one | Calcd. 524.1, found 523.9 |
| **31** | 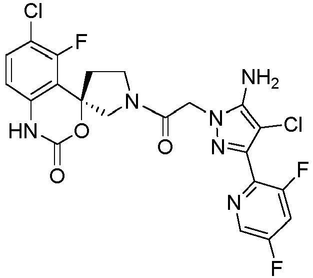 | (*R*)-1'-(2-(5-Amino-4-chloro-3-(3,5-difluoropyridin-2-yl)-1*H-*pyrazol-1-yl)acetyl)-6-chloro-5-fluorospiro[benzo[*d*][1,3]oxazine-4,3'-pyrrolidin]-2(1*H*)-one | Calcd. 527.1, found 527.0 |
| **32** | 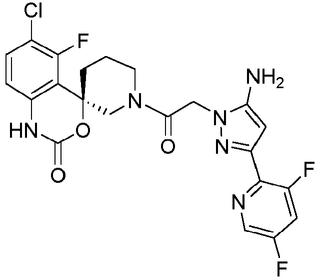 | (*R*)-1'-(2-(5-Amino-3-(3,5-difluoropyridin-2-yl)-1*H*-pyrazol-1-yl)acetyl)-6-chloro-5-fluorospiro[benzo[*d*][1,3]oxazine-4,3'-piperidin]-2(1*H*)-one | Calcd. 507.1, found 507.0 |
| **33** | 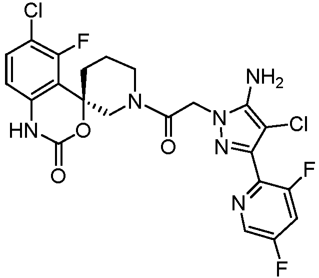 | (*R*)-1'-(2-(5-Amino-4-chloro-3-(3,5-difluoropyridin-2-yl)-1*H-*pyrazol-1-yl)acetyl)-6-chloro-5-fluorospiro[benzo[*d*][1,3]oxazine-4,3'-piperidin]-2(1*H*)-one | Calcd. 541.1, found 540.8 |
| **34** | 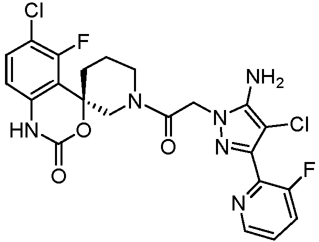 | (*R*)-1'-(2-(5-Amino-4-chloro-3-(3-fluoropyridin-2-yl)-1*H-*pyrazol-1-yl)acetyl)-6-chloro-5-fluorospiro[benzo[*d*][1,3]oxazine-4,3'-piperidin]-2(1*H*)-one | Calcd. 523.1, found 523.0 |
| **35** | 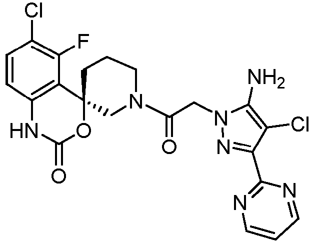 | (*R*)-1'-(2-(5-Amino-4-chloro-3-(pyrimidin-2-yl)-1*H*-pyrazol-1-yl)acetyl)-6-chloro-5-fluorospiro[benzo[*d*][1,3]oxazine-4,3'-piperidin]-2(1*H*)-one | Calcd. 506.1, found 505.9 |
| **36** | 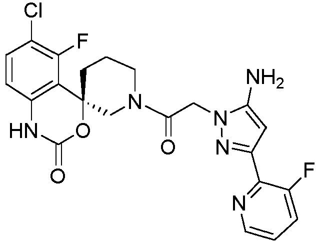 | (*R*)-1'-(2-(5-Amino-3-(3-fluoropyridin-2-yl)-1*H*-pyrazol-1-yl)acetyl)-6-chloro-5-fluorospiro[benzo[*d*][1,3]oxazine-4,3'-piperidin]-2(1*H*)-one | Calcd. 489.1, found 489.0 |

### Example 37

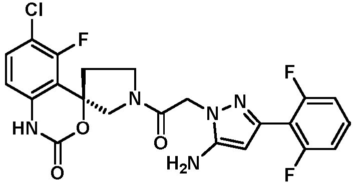

### (R)-1'-(2-(5-Amino-3-(2,6-difluorophenyl)-1H-pyrazol-1-yl)acetyl)-6-chloro-5-fluorospiro[benzo[d][1,3]oxazine-4,3'-pyrrolidin]-2(1H)-one

A mixture of **i-5** (25 mg, 0.056 mmol) and 3-(2,6-difluorophenyl)-3-oxopropanenitrile (10 mg, 0.056 mmol) in MeOH (0.6 mL) was heated in a microwave reactor at 100 °C for 1 h. The reaction was cooled to rt and purified by reverse phase HPLC (ACN/water + 0.1% TFA) to obtain the title compound. ¹H NMR (500 MHz, DMSO-*d₆*) δ 10.76 (s, 1H), 7.58 (dt, *J* = 12.7, 8.3 Hz, 1H), 7.41 (ddt, *J* = 12.2, 9.7, 4.6 Hz, 1H), 7.20 - 7.11 (m, 2H), 6.81 (t, *J* = 9.2 Hz, 1H), 5.66 (d, *J* = 12.9 Hz, 1H), 5.05 - 4.85 (m, 2H), 4.83 (br, 2H), 4.18 - 3.52 (m, 4H), 2.65 -2.47 (m, 2H). LCMS [M+H]⁺ = 492.2 (Calcd. 492.1).

The following compounds were prepared using procedures similar to those described in **Example 37** using the appropriate starting materials.

| **Example No.** | **Structure** | **Name** | **LCMS [M+H]⁺** |
|---|---|---|---|
| **38** | 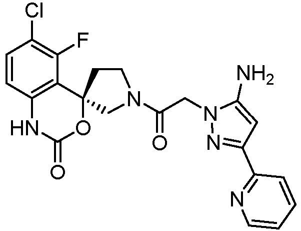 | (*R*)-1'-(2-(5-Amino-3-(pyridin-2-yl)-1*H*-pyrazol-1-yl)acetyl)-6-chloro-5-fluorospiro[benzo[*d*][1,3]o xazine-4,3'-pyrrolidin]-2(1*H*)-one | Calcd. 457.1, found 457.2 |
| **39** | 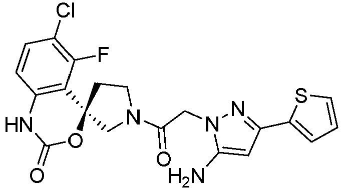 | (*R*)-1'-(2-(5-Amino-3-(thiophen-2-yl)-1*H-*pyrazol-1-yl)acetyl)-6-chloro-5-fluorospiro[benzo[d][1,3]o xazine-4,3'-pyrrolidin]-2(1*H*)-one | Calcd. 462.1, found 461.9 |
| **40** | 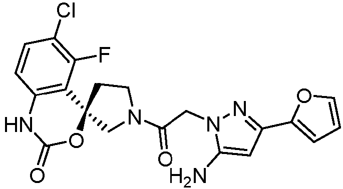 | (*R*)-1'-(2-(5-Amino-3-(furan-2-yl)-1*H*-pyrazol-1-yl)acetyl)-6-chloro-5-fluorospiro[benzo[*d*][1,3]o xazine-4,3'-pyrrolidin]-2(1*H*)-one | Calcd. 446.1, found 445.9 |
| **41** | 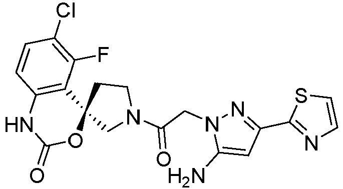 | (*R*)-1'-(2-(5-Amino-3-(thiazol-2-yl)-1*H*-pyrazol-1-yl)acetyl)-6-chloro-5-fluorospiro[benzo[*d*][1,3]o xazine-4,3'-pyrrolidin]-2(1*H*)-one | Calcd. 463.1, found 462.9 |
| **42** | 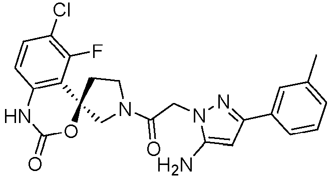 | (*R*)-1'-(2-(5-Amino-3-(m-tolyl)-1*H*-pyrazol-1-yl)acetyl)-6-chloro-5-fluorospiro[benzo[*d*][1,3]o xazine-4,3'-pyrrolidin]-2(1*H*)-one | Calcd. 470.1, found 470.0 |
| **43** | 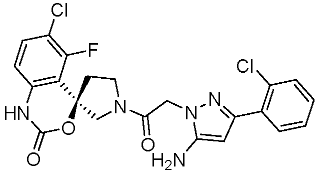 | (*R*)-1'-(2-(5-Amino-3-(2-chlorophenyl)-1*H*-pyrazol-1-yl)acetyl)-6-chloro-5-fluorospiro[benzo[*d*][1,3]o xazine-4,3'-pyrrolidin]-2(1*H*)-one | Calcd. 490.1, found 489.9 |
| **44** | 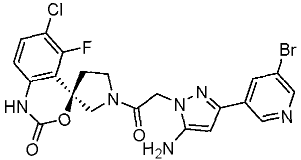 | (*R*)-1'-(2-(5-Amino-3-(5-bromopyridin-3-yl)-1*H-*pyrazol-1-yl)acetyl)-6-chloro-5-fluorospiro[benzo[*d*][1,3]o xazine-4,3'-pyrrolidin]-2(1*H*)-one | Calcd. 535.0, found 534.8 |
| **45** | 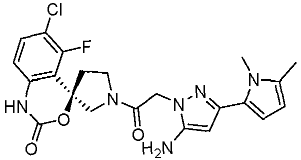 | (*R*)-1'-(2-(5-Amino-3-(1,5-dimethyl-1*H*-pyrrol-2-yl)-1*H*-pyrazol-1-yl)acetyl)-6-chloro-5-fluorospiro[benzo[*d*][1,3]o xazine-4,3'-pyrrolidin]-2(1*H*)-one | Calcd. 473.1, found 472.9 |
| **46** | 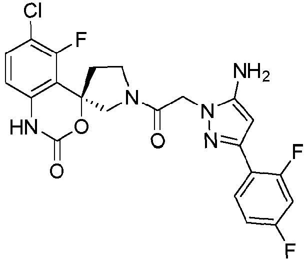 | (*R*)-1'-(2-(5-Amino-3-(2,4-difluorophenyl)-1*H-*pyrazol-1-yl)acetyl)-6-chloro-5-fluorospiro[benzo[*d*][1,3]o xazine-4,3'-pyrrolidin]-2(1*H*)-one | Calcd. 492.1, found 492.2 |
| **47** | 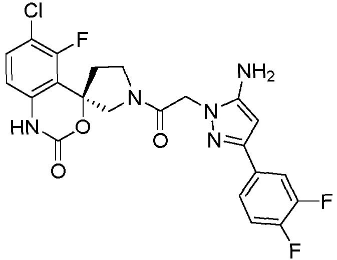 | (*R*)-1'-(2-(5-Amino-3-(3,4-difluorophenyl)-1*H-*pyrazol-1-yl)acetyl)-6-chloro-5-fluorospiro[benzo[*d*][1,3]o xazine-4,3'-pyrrolidin]-2(1*H*)-one | Calcd. 492.1, found 492.4 |
| **48** | 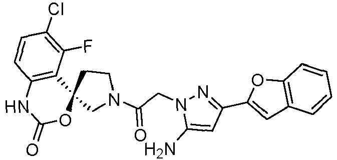 | (*R*)-1'-(2-(5-Amino-3-(benzofuran-2-yl)-1*H-*pyrazol-1-yl)acetyl)-6-chloro-5-fluorospiro[benzo[*d*][1,3]o xazine-4,3'-pyrrolidin]-2(1*H*)-one | Calcd. 496.1, found 496.1 |
| **49** | 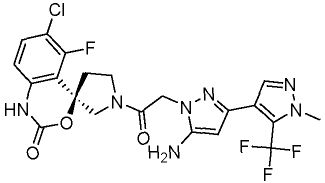 | (*R*)-1'-(2-(5-Amino-1'-methyl-5'-(trifluoromethyl)-1*H*,1'*H-*[3,4'-bipyrazol]-1-yl)acetyl)-6-chloro-5-fluorospiro[benzo[*d*][1,3]o xazine-4,3'-pyrrolidin]-2(1*H*)-one | Calcd. 528.1, found 528.1 |
| **50** | 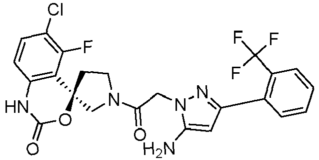 | (*R*)-1'-(2-(5-Amino-3-(2-(trifluoromethyl)phenyl)-1*H*-pyrazol-1-yl)acetyl)-6-chloro-5-fluorospiro[benzo[*d*][1,3]o xazine-4,3'-pyrrolidin]-2(1*H*)-one | Calcd. 524.1, found 524.1 |
| **51** | 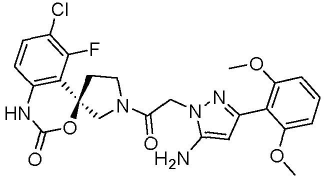 | (*R*)-1'-(2-(5-Amino-3-(2,6-dimethoxyphenyl)-1*H-*pyrazol-1-yl)acetyl)-6-chloro-5-fluorospiro[benzo[*d*][1,3]o xazine-4,3'-pyrrolidin]-2(1*H*)-one | Calcd. 516.1, found 516.1 |
| **52** | 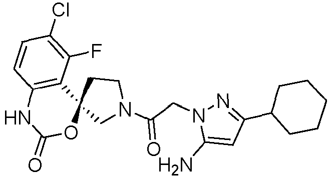 | (*R*)-1'-(2-(5-Amino-3-cyclohexyl-1*H*-pyrazol-1-yl)acetyl)-6-chloro-5-fluorospiro[benzo[*d*][1,3]o xazine-4,3'-pyrrolidin]-2(1*H*)-one | Calcd. 462.2, found 462.2 |
| **53** | 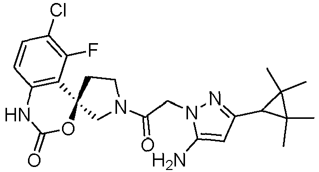 | (*R*)-1'-(2-(5-Amino-3-(2,2,3,3-tetramethylcyclopropyl)-1*H*-pyrazol-1-yl)acetyl)-6-chloro-5-fluorospiro[benzo[*d*][1,3]o xazine-4,3'-pyrrolidin]-2(1*H*)-one | Calcd. 476.2, found 476.2 |
| **54** | 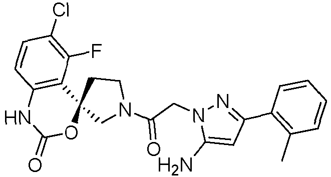 | (*R*)-1'-(2-(5-Amino-3-(o-tolyl)-1*H*-pyrazol-1-yl)acetyl)-6-chloro-5-fluorospiro[benzo[*d*][1,3]o xazine-4,3'-pyrrolidin]-2(1*H*)-one | Calcd. 470.1, found 470.1 |
| **55** | 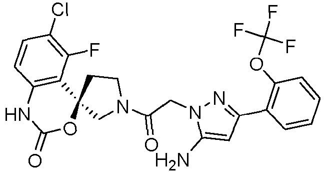 | (*R*)-1'-(2-(5-Amino-3-(2-(trifluoromethoxy)phenyl)-1*H*-pyrazol-1-yl)acetyl)-6-chloro-5-fluorospiro[benzo[*d*][1,3]o xazine-4,3'-pyrrolidin]-2(1*H*)-one | Calcd. 540.1, found 540.1 |
| **56** | 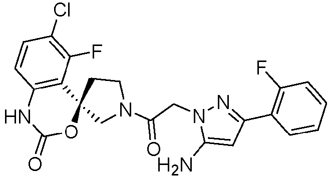 | (*R*)-1'-(2-(5-Amino-3-(2-fluorophenyl)-1*H*-pyrazol-1-yl)acetyl)-6-chloro-5-fluorospiro[benzo[*d*][1,3]o xazine-4,3'-pyrrolidin]-2(1*H*)-one | Calcd. 474.1, found 474.1 |
| **57** | 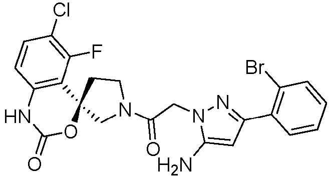 | (*R*)-1'-(2-(5-Amino-3-(2-bromophenyl)-1*H*-pyrazol-1-yl)acetyl)-6-chloro-5-fluorospiro[benzo[*d*][1,3]o xazine-4,3'-pyrrolidin]-2(1*H*)-one | Calcd. 534.0, found 534.0 |
| **58** | 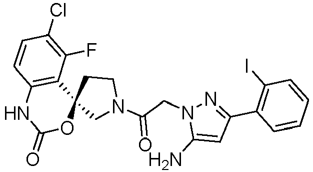 | (*R*)-1'-(2-(5-Amino-3-(2-iodophenyl)-1*H*-pyrazol-1-yl)acetyl)-6-chloro-5-fluorospiro[benzo[*d*][1,3]o xazine-4,3'-pyrrolidin]-2(1*H*)-one | Calcd. 582.0, found 582.0 |
| **59** | 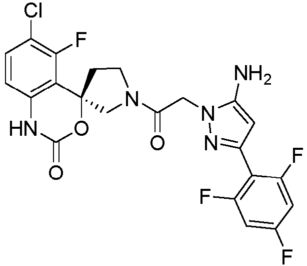 | (*R*)-1'-(2-(5-Amino-3-(2,4,6-trifluorophenyl)-1*H-*pyrazol-1-yl)acetyl)-6-chloro-5-fluorospiro[benzo[*d*][1,3]o xazine-4,3'-pyrrolidin]-2(1*H*)-one | Calcd. 510.1, found 510.2 |
| **60** | 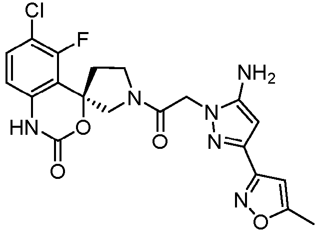 | (*R*)-1'-(2-(5-Amino-3-(5-methylisoxazol-3-yl)-1*H-*pyrazol-1-yl)acetyl)-6-chloro-5-fluorospiro[benzo[*d*][1,3]o xazine-4,3'-pyrrolidin]-2(1*H*)-one | Calcd. 461.1, found 461.2 |
| **61** | 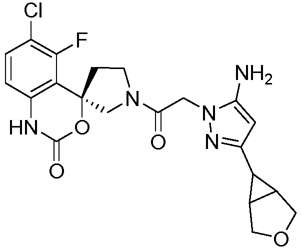 | (4*R*)-1'-(2-(5-Amino-3-(3-oxabicyclo[3.1.0]hexan-6-yl)-1*H*-pyrazol-1-yl)acetyl)-6-chloro-5-fluorospiro[benzo[*d*][1,3]o xazine-4,3'-pyrrolidin]-2(1*H*)-one | Calcd. 462.1, found 462.0 |
| **62** | 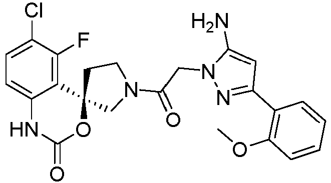 | (*R*)-1'-(2-(5-Amino-3-(2-methoxyphenyl)-1*H-*pyrazol-1-yl)acetyl)-6-chloro-5-fluorospiro[benzo[*d*][1,3]o xazine-4,3'-pyrrolidin]-2(1*H*)-one | Calcd. 486.1, found 486.1 |

### Example 63

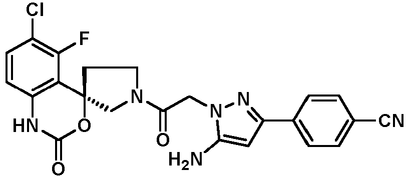

### (R)-4-(5-Amino-1-(2-(6-chloro-5-fluoro-2-oxo-1,2-dihydrospiro[benzo[d][1,3]oxazine-4,3'-pyrrolidin]-1'-yl)-2-oxoethyl)-1H-pyrazol-3-yl)benzonitrile

**i-6** (80 mg, 0.11 mmol), (4-cyanophenyl)boronic acid (22 mg, 0.15 mmol), and Pd(OAc)₂ (3.6 mg, 0.016 mmol) were combined in a microwave vial, capped and degassed. Degassed ethanol (0.53 mL) and water (0.53 mL) were added, followed by DBU (22 µL, 0.15 mmol), and the resulting mixture was degassed again. The reaction mixture was heated in a microwave reactor at 150 °C for 10 min. The reaction was cooled to rt, diluted with EtOH, filtered and concentrated to afford a crude residue that was purified by preparative reverse phase HPLC (ACN/water + 0.1% TFA to give the title compound. ¹H NMR (500 MHz, DMSO-*d₆*) δ 10.81 (s, 1H), 10.77 (s, 1H), 7.87 (t, *J* = 8.2 Hz, 2H), 7.80 (dd, *J* = 8.4, 2.0 Hz, 2H), 7.58 (dt, *J* = 15.6, 8.3 Hz, 1H), 6.82 (dd, *J =* 12.4, 8.9 Hz, 1H), 5.86 (d, *J* = 6.3 Hz, 1H), 4.98 4.82 (m, 2H), 4.21 -3.76 (m, 4H), 2.61-2.51 (m, 2H). LCMS [M+H]⁺ = 481.2 (Calcd. 481.1).

The following compounds were prepared using procedures similar to those described in **Example 63** using the appropriate starting materials.

| **Example No.** | **Structure** | **Name** | **LCMS [M+H]⁺** |
|---|---|---|---|
| **64** | 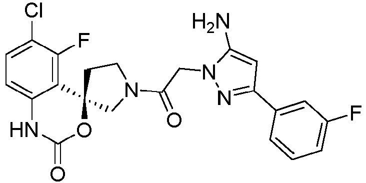 | (*R*)-1'-(2-(5-Amino-3-(3-fluorophenyl)-1*H-*pyrazol-1-yl)acetyl)-6-chloro-5-fluorospiro[benzo[*d*][1, 3]oxazine-4,3'-pyrrolidin]-2(1*H*)-one | Calcd. 474.1, found 474.1 |
| **65** | 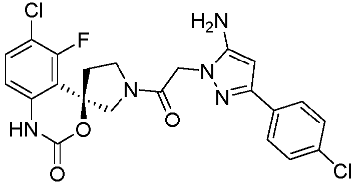 | (*R*)-1'-(2-(5-Amino-3-(4-chlorophenyl)-1*H-*pyrazol-1-yl)acetyl)-6-chloro-5-fluorospiro[benzo[*d*][1, 3]oxazine-4,3'-pyrrolidin]-2(1*H*)-one | Calcd. 490.1, found 490.1 |
| **66** | 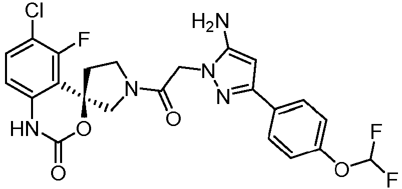 | (*R*)-1'-(2-(5-Amino-3-(4-(difluoromethoxy)phen yl)-1*H*-pyrazol-1-yl)acetyl)-6-chloro-5-fluorospiro[benzo[*d*][1, 3]oxazine-4,3'-pyrrolidin]-2(1*H*)-one | Calcd. 522.1, found 522.1 |
| **67** | 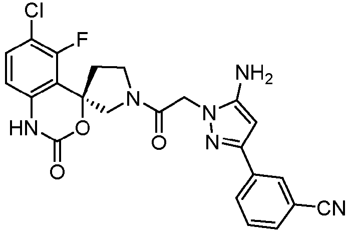 | *(R*)-3-(5-Amino-1-(2-(6-chloro-5-fluoro-2-oxo-1,2-dihydrospiro[benzo[*d*][ 1,3]oxazine-4,3'-pyrrolidin]-1'-yl)-2-oxoethyl)-1*H*-pyrazol-3-yl)benzonitrile | Calcd. 481.1, found 480.9 |

### Plasma Kallikrein assay

The effectiveness of a compound of the present invention as an inhibitor of plasma kallikrein can be determined using a relevant purified serine protease, and an appropriate synthetic substrate. The rate of hydrolysis of the chromogenic or fluorogenic substrate by the relevant serine protease was measured both in the absence and presence of compounds of the present invention. Assays were conducted at rt or at 37 °C. Hydrolysis of the substrate resulted in release of amino trifluoromethylcoumarin (AFC), which was monitored spectrofluorometrically by measuring the increase in emission at 510 nm with excitation at 405 nm. A decrease in the rate of fluorescence change in the presence of inhibitor is indicative of enzyme inhibition. Such methods are known to one skilled in the art. The results of this assay are expressed as the half-maximal inhibitory concentrations (IC₅₀), or the inhibitory constant, Kᵢ.

Plasma kallikrein determinations were made in 50 mM HEPES buffer at pH 7.4 containing 150 mM NaCl, 5 mM CaCl₂, and 0.1% PEG 8000 (polyethylene glycol; Fisher Scientific). Determinations were made using purified Human plasma kallikrein at a final concentration of 0.5 nM (Enzyme Research Laboratories) and the synthetic substrate, Acetyl-K-P-R-AFC (Sigma # C6608) at a concentration of 100 mM.

Activity assays were performed by diluting a stock solution of substrate at least tenfold to a final concentration ≤ 0.2 Km into a solution containing enzyme or enzyme equilibrated with inhibitor. Times required to achieve equilibration between enzyme and inhibitor were determined in control experiments. The reactions were performed under linear progress curve conditions and fluorescence increase measured at 405 Ex/510 Em nm. Values were converted to percent inhibition of the control reaction (after subtracting 100% Inhibition value). IC₅₀ was determined by inflection point from a four parameter logistic curve fit. Ki was calculated using the Cheng Prusoff equation, Ki = IC₅₀/(1+([S]/Km)).

The activities shown by this assay indicate that the compounds of the invention may be therapeutically useful for treating or preventing various ophthalmic, cardiovascular and/or cerebrovascular thromboembolic conditions in patients suffering from unstable angina, acute coronary syndrome, refractory angina, myocardial infarction, transient ischemic attacks, atrial fibrillation, stroke such as thrombotic stroke or embolic stroke, venous thrombosis, coronary and cerebral arterial thrombosis, cerebral and pulmonary embolism, atherosclerosis, deep vein thrombosis, disseminated intravascular coagulation, reocclusion or restenosis of recanalized vessels, hereditary angioedema, uveitis, posterior uveitis, wet age-related macular degeneration, diabetic macular edema, diabetic retinopathy and retinal vein occlusion.

### Factor XIa assay

The effectiveness of a compound of the present invention as an inhibitor of Coagulation factor XIa can be determined using a relevant purified serine protease, and an appropriate synthetic substrate. The rate of hydrolysis of the chromogenic or fluorogenic substrate by the relevant serine protease was measured both in the absence and presence of compounds of the present invention. Assays were conducted at rt or at 37 °C. Hydrolysis of the substrate resulted in release of amino trifluoromethylcoumarin (AFC), which was monitored spectrofluorometrically by measuring the increase in emission at 510 nm with excitation at 405 nm. A decrease in the rate of fluorescence change in the presence of inhibitor is indicative of enzyme inhibition. Such methods are known to one skilled in the art. The results of this assay are expressed as the half-maximal inhibitory concentrations (IC₅₀), or the inhibitory constant, Kᵢ.

Compounds were pre-incubated for 30 min at 25 °C with human (0.04 nM) factor XIa in 50 mM HEPES buffer with 150 mM sodium chloride, 5 mM calcium chloride, 0.1% PEG 8000, pH 7.4. factor XIa enzymatic activity was determined by addition of the substrate glycine-proline-arginine-7-amido-4-trifluoromethylcoumarin (GPR-AFC) and measurement of the fluorescence at 400/505 nm after a 60 min incubation at 25 °C. The % inhibition for each data point was calculated from the data and analyzed using the log (inhibitor) vs. response four parameters equation to determine the half-maximal inhibitory concentrations (IC₅₀). The IC₅₀ were converted to equilibrium inhibitory constants (Ki) using the Cheng-Prusoff equation.

The activities shown by this assay indicate that the compounds of the invention may be therapeutically useful for treating or preventing various cardiovascular and/or cerebrovascular thromboembolic conditions in patients suffering from unstable angina, acute coronary syndrome, refractory angina, myocardial infarction, transient ischemic attacks, atrial fibrillation, stroke such as thrombotic stroke or embolic stroke, venous thrombosis, coronary and cerebral arterial thrombosis, cerebral and pulmonary embolism, atherosclerosis, deep vein thrombosis, disseminated intravascular coagulation, and reocclusion or restenosis of recanalized vessels.

Plasma Kallikrein (PKal) IC₅₀ (nM) and Factor XIa (FXIa) IC₅₀ (nM) for selected compounds are as follows:

| **Example** | PKal IC₅₀ (nM) | FXIa IC₅₀ (nM) |
|---|---|---|
| **1** | 9.9 | 513 |
| **2** | 8.0 | 653 |
| **3** | 37.8 | 347 |
| **4** | 16.3 | 315 |
| **5** | 30.6 | 685 |
| **6** | 10.1 | 904 |
| **7** | 49.0 | 4136 |
| **8** | 11.8 | 679 |
| **9** | 12.9 | 389 |
| **10** | 27.4 | 506 |
| **11** | 32.3 | 672 |
| **12** | 37.5 | 2709 |
| **13** | 38.8 | 277 |
| **14** | 3.3 | 29 |
| **15** | 40.6 | 100 |
| **16** | 20.4 | 2823 |
| **17** | 21.0 | 2552 |
| **18** | 2.4 | 88 |
| **19** | 30.3 | 134 |
| **20** | 42.3 | 214 |
| **21** | 19.0 | 325 |
| **22** | 26.3 | 516 |
| **23** | 11.8 | 1790 |
| **24** | 15.4 | 114 |
| **25** | 12.7 | 367 |
| **26** | 22.2 | 484 |
| **27** | 25.3 | 424 |
| **28** | 16.7 | 55 |
| **29** | 32.3 | 1246 |
| **30** | 37.4 | 164 |
| **31** | 32.1 | 1831 |
| **32** | 5.7 | 648 |
| **33** | 7.7 | 187 |
| **34** | 8.9 | 177 |
| **35** | 24.5 | 280 |
| **36** | 8.4 | 826 |
| **37** | 2.2 | 102 |
| **38** | 28.0 | 665 |
| **39** | 13.0 | 254 |
| **40** | 16.2 | 225 |
| **41** | 18.1 | 286 |
| **42** | 15.1 | 611 |
| **43** | 3.2 | 187 |
| **44** | 11.2 | 389 |
| **45** | 40.3 | 2762 |
| **46** | 5.0 | 263 |
| **47** | 14.3 | 739 |
| **48** | 7.8 | 1399 |
| **49** | 1.4 | 184 |
| **50** | 5.4 | 422 |
| **51** | 23.5 | 1767 |
| **52** | 20.7 | 426 |
| **53** | 11.5 | 320 |
| **54** | 5.1 | 289 |
| **55** | 2.5 | 287 |
| **56** | 4.6 | 165 |
| **57** | 2.5 | 190 |
| **58** | 5.7 | 430 |
| **59** | 3.7 | 195 |
| **60** | 24.3 | 862 |
| **61** | 27.2 | 791 |
| **62** | 6.6 | 505 |
| **63** | 13.9 | 613 |
| **64** | 17.0 | 556 |
| **65** | 21.9 | 1563 |
| **66** | 10.3 | 640 |
| **67** | 12.0 | 433 |

## Claims

1. A compound of the Formula I: 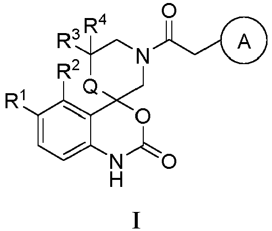 wherein 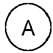 is 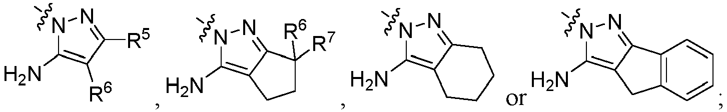
Q is -CH₂- or absent;
R¹ is selected from the group consisting of hydrogen, halo, hydroxy and C₁₋₆ alkyl;
R² is selected from the group consisting of hydrogen, halo, hydroxy and C₁₋₆ alkyl;
R³ is selected from the group consisting of hydrogen, halo, hydroxy and C₁₋₆ alkyl;
R⁴ is selected from the group consisting of hydrogen, halo, hydroxy and C₁₋₆ alkyl;
R⁵ is selected from the group consisting of
(a) phenyl, which is optionally substituted with one to three substituents selected from the group consisting of halo, cyano, R^{×} and OR^{x};
(b) C₃₋₆ cycloalkyl, which is optionally substituted with one to four substituents selected from the group consisting of halo and R^{×};
(c) heteroaryl, which can be monocyclic or bicyclic, which is optionally substituted with one or two substituents selected from the group consisting of halo and R^{×}; and
(d) heterocyclyl, which can be monocyclic or bicyclic;
R⁶ is selected from the group consisting of hydrogen, halo, hydroxy and C₁₋₆ alkyl;
R⁷ is selected from the group consisting of hydrogen, halo, hydroxy and C₁₋₆ alkyl;
R^{x} is hydrogen or C₁₋₆ alkyl, which is optionally substituted with one to three substituents selected from the group consisting of halo and hydroxy;
or a pharmaceutically acceptable salt thereof.

2. The compound of Claim 1 wherein Q is -CH₂-, or a pharmaceutically acceptable salt thereof.

3. The compound of Claims 1 or 2 of the Formula Ia: 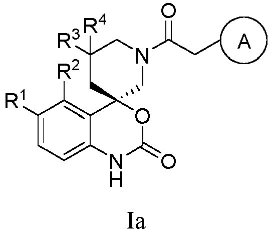 wherein 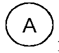
is 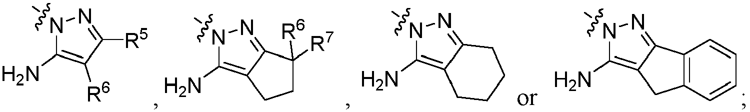
R¹ is selected from the group consisting of hydrogen, halo, hydroxy and C₁₋₆ alkyl;
R² is selected from the group consisting of hydrogen, halo, hydroxy and C₁₋₆ alkyl;
R³ is selected from the group consisting of hydrogen, halo, hydroxy and C₁₋₆ alkyl;
R⁴ is selected from the group consisting of hydrogen, halo, hydroxy and C₁₋₆ alkyl;
R⁵ is selected from the group consisting of
(a) phenyl, which is optionally substituted with one to three substituents selected from the group consisting of halo, cyano, R^{x} and OR^{x};
(b) C₃₋₆ cycloalkyl, which is optionally substituted with one to four substituents selected from the group consisting of halo and R^{×};
(c) heteroaryl, which can be monocyclic or bicyclic, which is optionally substituted with one or two substituents selected from the group consisting of halo and R^{×}; and
(d) heterocyclyl, which can be monocyclic or bicyclic;
R⁶ is selected from the group consisting of hydrogen, halo, hydroxy and C₁₋₆ alkyl;
R⁷ is selected from the group consisting of hydrogen, halo, hydroxy and C₁₋₆ alkyl;
R^{x} is hydrogen or C₁₋₆ alkyl, which is optionally substituted with one to three substituents selected from the group consisting of halo and hydroxy;
or a pharmaceutically acceptable salt thereof.

4. The compound of Claim 1 of Formula 1b 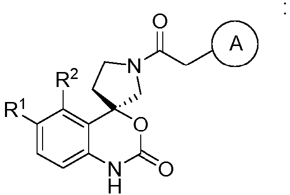 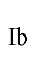 wherein 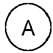 is 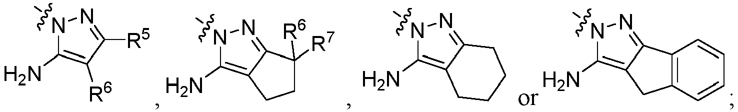
R¹ is selected from the group consisting of hydrogen, halo, hydroxy and C₁₋₆ alkyl;
R² is selected from the group consisting of hydrogen, halo, hydroxy and C₁₋₆ alkyl;
R⁵ is selected from the group consisting of
(a) phenyl, which is optionally substituted with one to three substituents selected from the group consisting of halo, cyano, R^{x} and OR^{x};
(b) C₃₋₆ cycloalkyl, which is optionally substituted with one to four substituents selected from the group consisting of halo and R^{x};
(c) heteroaryl, which can be monocyclic or bicyclic, which is optionally substituted with one or two substituents selected from the group consisting of halo and R^{×}; and
(d) heterocyclyl, which can be monocyclic or bicyclic;
R⁶ is selected from the group consisting of hydrogen, halo, hydroxy and C₁₋₆ alkyl;
R⁷ is selected from the group consisting of hydrogen, halo, hydroxy and C₁₋₆ alkyl;
R^{x} is hydrogen or C₁₋₆ alkyl, which is optionally substituted with one to three substituents selected from the group consisting of halo and hydroxy,
or a pharmaceutically acceptable salt thereof.

5. The compound of any of Claims 1 to 4 wherein R¹ is halo and R² is halo, or a pharmaceutically acceptable salt thereof.

6. The compound of any of Claims 1, 2, 3 or 5 wherein R³ is hydrogen and R⁴ is hydrogen, or a pharmaceutically acceptable salt thereof.

7. The compound of any of Claims 1 to 6 wherein R⁵ is selected from the group consisting of
(a) phenyl, which is optionally substituted with one to three substituents selected from the group consisting of halo, cyano, methyl, methoxy, difluoromethoxy, trifluoromethoxy and trifluoromethyl;
(b) cyclopropyl, cyclobutyl, cyclopentyl or cyclohexyl, wherein said cyclopropyl is optionally substituted with one to four substituents selected from the group consisting of methyl and halo;
(c) benzodioxyl, pyridinyl, pyridazinyl, pyrimidinyl, thiophenyl, furanyl, thiazolyl, pyrrolyl, benzofuranyl, pyrazolyl or isoxazolyl, wherein said pyridinyl, pyrazolyl, pyrrolyl and isoxazolyl groups are optionally substituted with one or two substituents selected from the group consisting of halo, methyl and trifluoromethyl; and
(d) tetrahydropyranyl or oxabicyclohexanyl;
or a pharmaceutically acceptable salt thereof.

8. The compound of any of Claims 1 to 7 wherein R⁵ is phenyl, which is optionally substituted with one to three substituents selected from the group consisting of halo, cyano, methyl, methoxy, difluoromethoxy, trifluoromethoxy and trifluoromethyl; or a pharmaceutically acceptable salt thereof.

9. The compound of Claim 1 selected from: 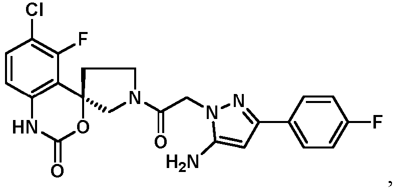 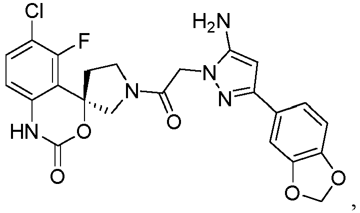 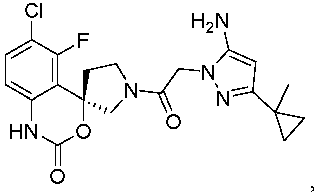 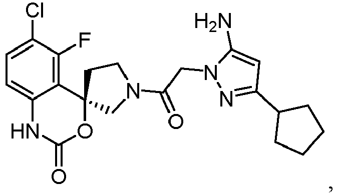 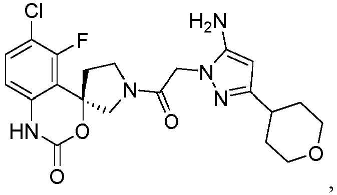 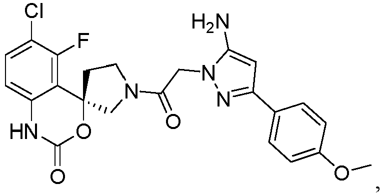 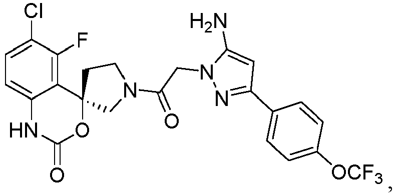 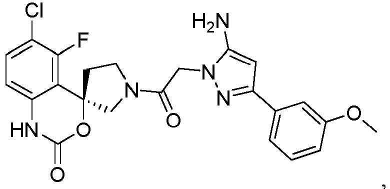 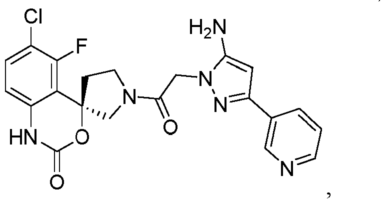 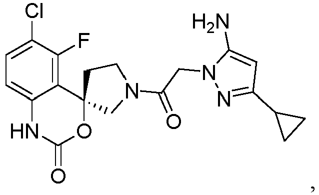 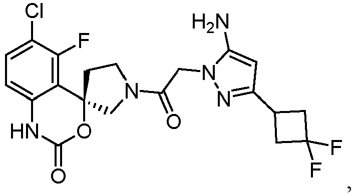 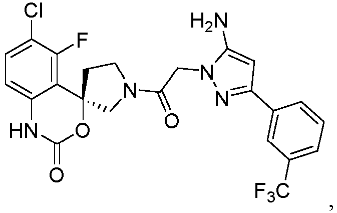 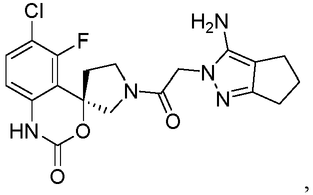 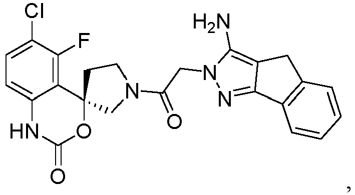 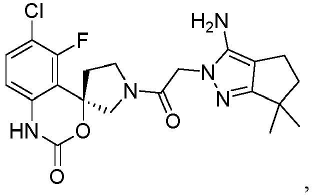 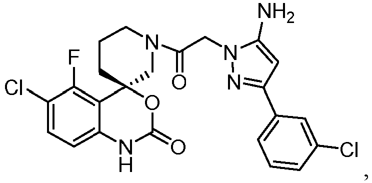 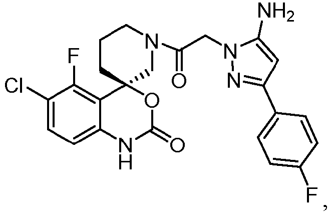 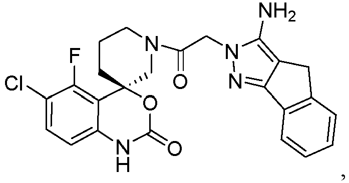 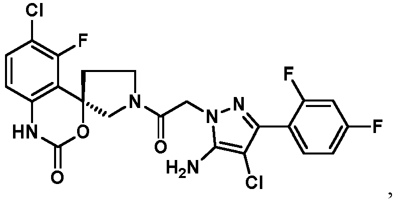 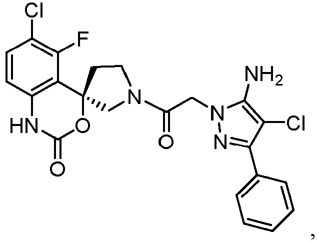 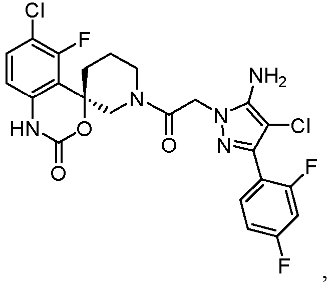 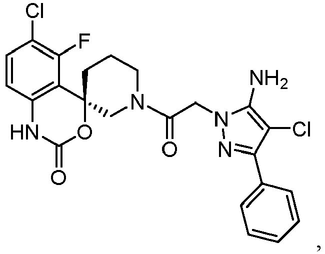 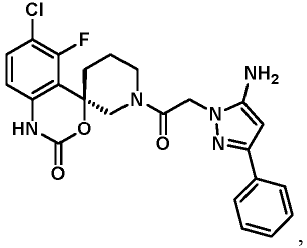 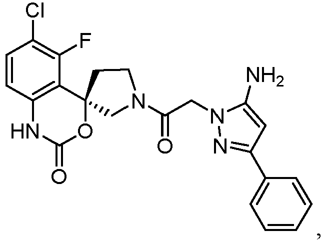 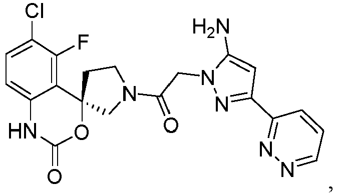 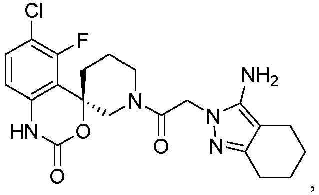 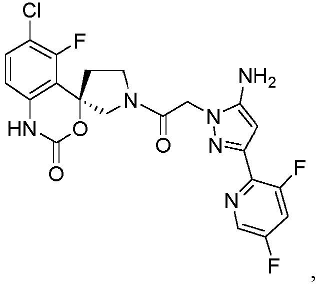 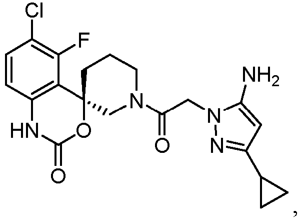 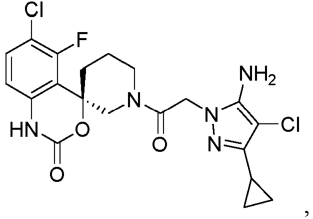 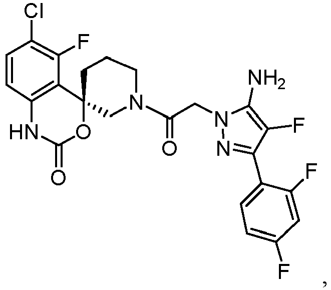 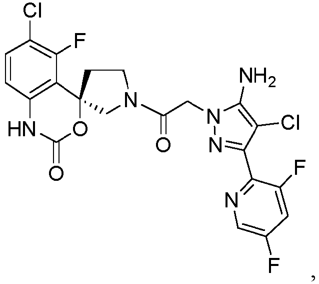 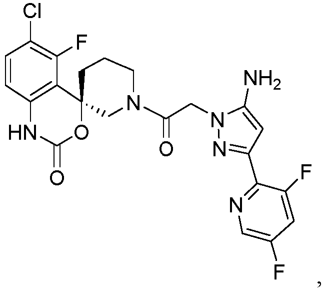 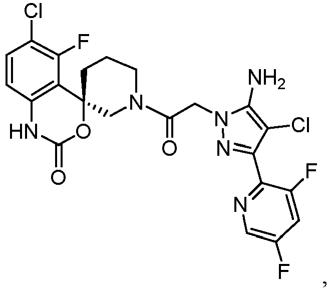 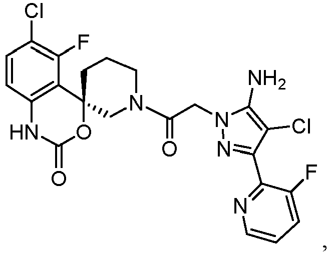 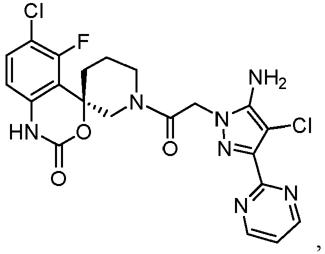 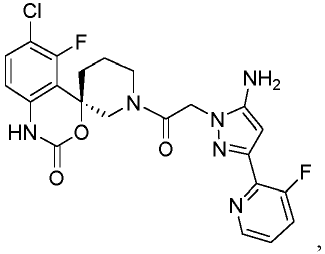 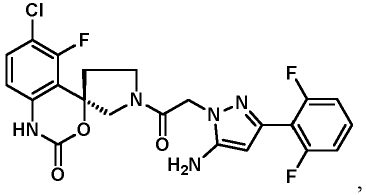 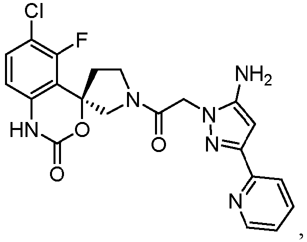 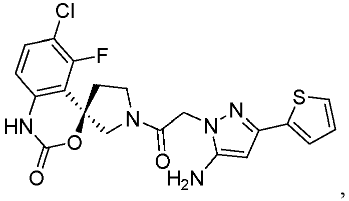 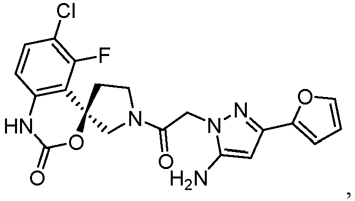 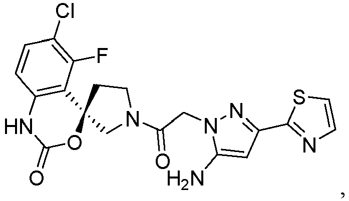 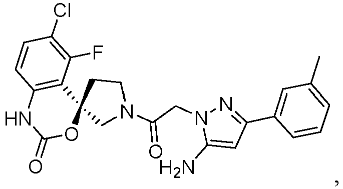 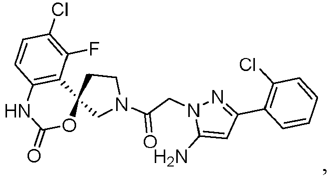 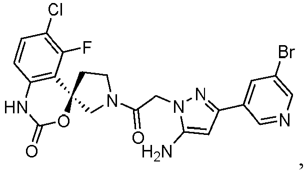 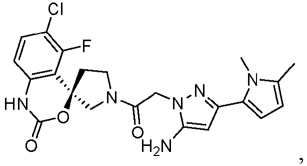 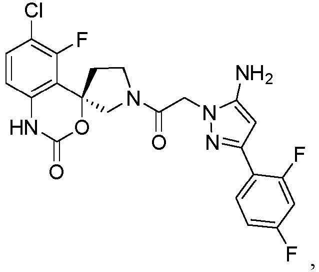 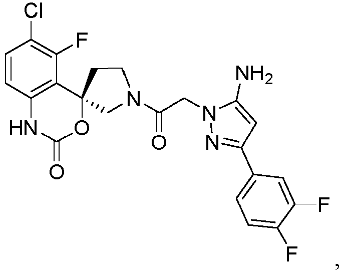 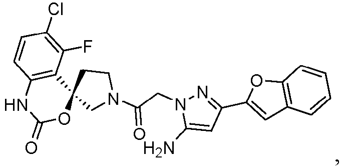 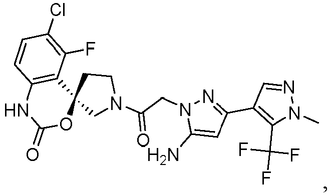 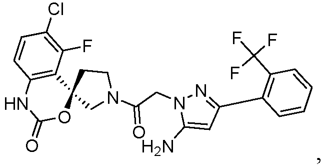 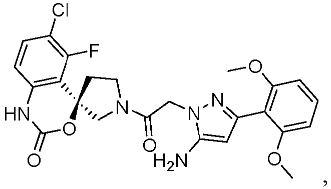 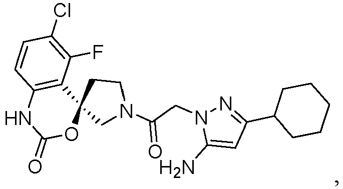 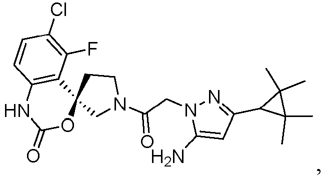 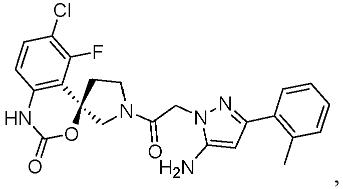 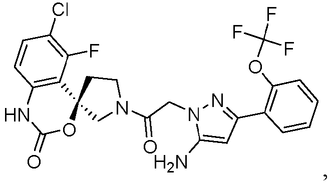 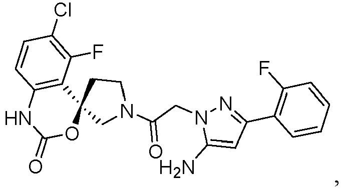 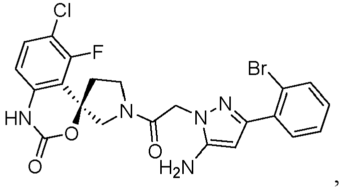 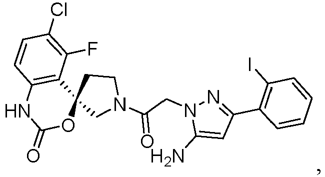 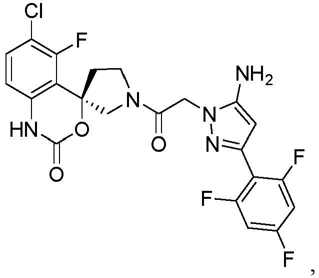 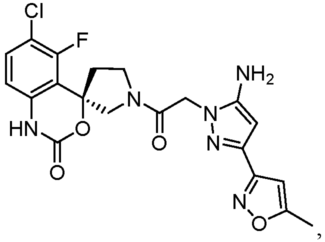 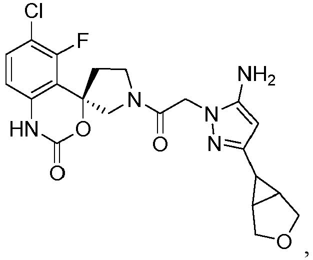 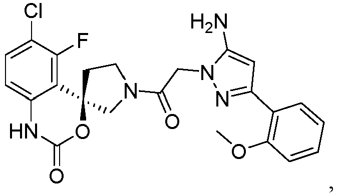 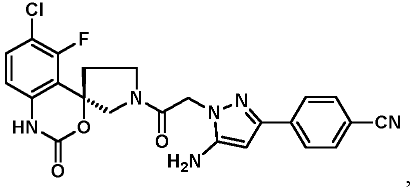 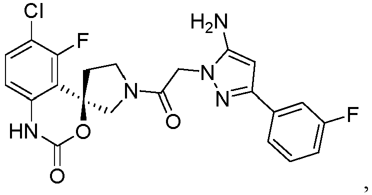 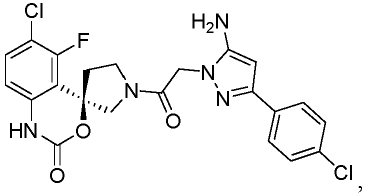 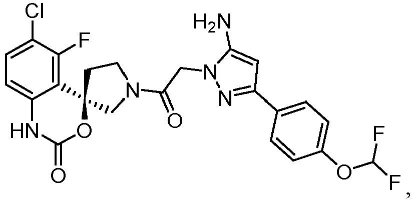 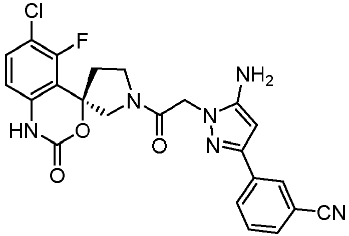 or a pharmaceutically acceptable salt thereof.

10. A pharmaceutical composition comprising a compound of any one of Claims 1 to 9 or a pharmaceutically acceptable salt thereof and a pharmaceutically acceptable carrier.

11. A compound of any of Claims 1 to 9, or a pharmaceutically acceptable salt thereof, or a pharmaceutical composition of Claim 10, for use in a method for treating impaired visual activity, diabetic retinopathy, diabetic macular edema, retinal vein occlusion, hereditary angioedema, diabetes, pancreatitis, cerebral hemorrhage, nephropathy, cardiomyopathy, neuropathy, inflammatory bowel disease, arthritis, inflammation, septic shock, hypotension, cancer, adult respiratory distress syndrome, disseminated intravascular coagulation, blood coagulation during cardiopulmonary bypass surgery, bleeding from postoperative surgery, uveitis, posterior uveitis, or wet age-related macular degeneration in a mammal.

12. The compound according to any one of Claims 1 to 9, or a pharmaceutically acceptable salt thereof, for use in therapy.

## Patentansprüche

1. Eine Verbindung der Formel I: 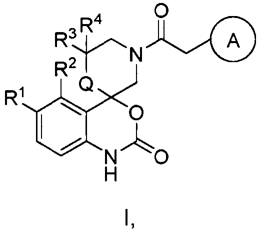 wobei 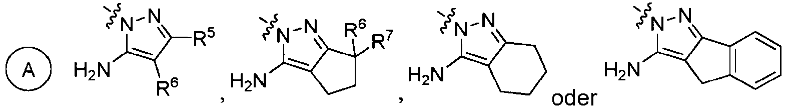 ist,
Q -CH₂- ist oder fehlt,
R¹ ausgewählt ist aus der Gruppe bestehend aus Wasserstoff, Halogen, Hydroxy und C₁₋₆-Alkyl,
R² ausgewählt ist aus der Gruppe bestehend aus Wasserstoff, Halogen, Hydroxy und C₁₋₆-Alkyl,
R³ ausgewählt ist aus der Gruppe bestehend aus Wasserstoff, Halogen, Hydroxy und C₁₋₆-Alkyl,
R⁴ ausgewählt ist aus der Gruppe bestehend aus Wasserstoff, Halogen, Hydroxy und C₁₋₆-Alkyl,
R⁵ ausgewählt ist aus der Gruppe bestehend aus
(a) Phenyl, das gegebenenfalls substituiert ist mit einem bis drei Substituenten, ausgewählt aus der Gruppe bestehend aus Halogen, Cyano, R^{x} und OR^{x},
(b) C₃₋₆-Cycloalkyl, das gegebenenfalls substituiert ist mit einem bis vier Substituenten, ausgewählt aus der Gruppe bestehend aus Halogen und R^{x},
(c) Heteroaryl, das monocyclisch oder bicyclisch sein kann, das gegebenenfalls substituiert ist mit einem oder zwei Substituenten, ausgewählt aus der Gruppe bestehend aus Halogen und R^{x}, und
(d) Heterocyclyl, das monocyclisch oder bicyclisch sein kann,
R⁶ ausgewählt ist aus der Gruppe bestehend aus Wasserstoff, Halogen, Hydroxy und C₁₋₆-Alkyl,
R⁷ ausgewählt ist aus der Gruppe bestehend aus Wasserstoff, Halogen, Hydroxy und C₁₋₆-Alkyl,
R^{×} Wasserstoff oder C₁₋₆-Alkyl ist, das gegebenenfalls substituiert ist mit einem bis drei Substituenten, ausgewählt aus der Gruppe bestehend aus Halogen und Hydroxy,
oder ein pharmazeutisch annehmbares Salz davon.

2. Die Verbindung nach Anspruch 1, wobei Q -CH₂- ist, oder ein pharmazeutisch annehmbares Salz davon.

3. Die Verbindung nach den Ansprüchen 1 oder 2 der Formel Ia: 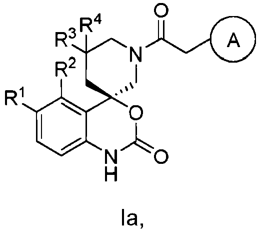 wobei 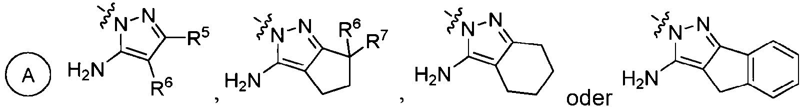 ist,
R¹ ausgewählt ist aus der Gruppe bestehend aus Wasserstoff, Halogen, Hydroxy und C₁₋₆-Alkyl,
R² ausgewählt ist aus der Gruppe bestehend aus Wasserstoff, Halogen, Hydroxy und C₁₋₆-Alkyl,
R³ ausgewählt ist aus der Gruppe bestehend aus Wasserstoff, Halogen, Hydroxy und C₁₋₆-Alkyl,
R⁴ ausgewählt ist aus der Gruppe bestehend aus Wasserstoff, Halogen, Hydroxy und C₁₋₆-Alkyl,
R⁵ ausgewählt ist aus der Gruppe bestehend aus
(a) Phenyl, das gegebenenfalls substituiert ist mit einem bis drei Substituenten, ausgewählt aus der Gruppe bestehend aus Halogen, Cyano, R^{×} und OR^{x},
(b) C₃₋₆-Cycloalkyl, das gegebenenfalls substituiert ist mit einem bis vier Substituenten, ausgewählt aus der Gruppe bestehend aus Halogen und R^{×},
(c) Heteroaryl, das monocyclisch oder bicyclisch sein kann, das gegebenenfalls substituiert ist mit einem oder zwei Substituenten, ausgewählt aus der Gruppe bestehend aus Halogen und R^{x}, und
(d) Heterocyclyl, das monocyclisch oder bicyclisch sein kann,
R⁶ ausgewählt ist aus der Gruppe bestehend aus Wasserstoff, Halogen, Hydroxy und C₁₋₆-Alkyl,
R⁷ ausgewählt ist aus der Gruppe bestehend aus Wasserstoff, Halogen, Hydroxy und C₁₋₆-Alkyl,
R^{×} Wasserstoff oder C₁₋₆-Alkyl ist, das gegebenenfalls substituiert ist mit einem bis drei Substituenten, ausgewählt aus der Gruppe bestehend aus Halogen und Hydroxy,
oder ein pharmazeutisch annehmbares Salz davon.

4. Die Verbindung nach Anspruch 1 der Formel Ib: 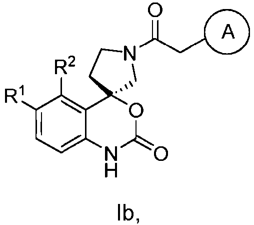 wobei 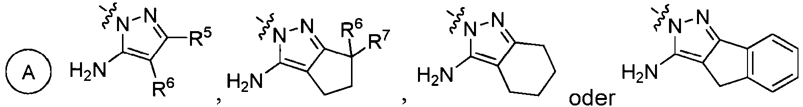 ist,
R¹ ausgewählt ist aus der Gruppe bestehend aus Wasserstoff, Halogen, Hydroxy und C₁₋₆-Alkyl,
R² ausgewählt ist aus der Gruppe bestehend aus Wasserstoff, Halogen, Hydroxy und C₁₋₆-Alkyl,
R⁵ ausgewählt ist aus der Gruppe bestehend aus
(a) Phenyl, das gegebenenfalls substituiert ist mit einem bis drei Substituenten, ausgewählt aus der Gruppe bestehend aus Halogen, Cyano, R^{x} und OR^{x},
(b) C₃₋₆-Cycloalkyl, das gegebenenfalls substituiert ist mit einem bis vier Substituenten, ausgewählt aus der Gruppe bestehend aus Halogen und R^{x},
(c) Heteroaryl, das monocyclisch oder bicyclisch sein kann, das gegebenenfalls substituiert ist mit einem oder zwei Substituenten, ausgewählt aus der Gruppe bestehend aus Halogen und R^{×}, und
(d) Heterocyclyl, das monocyclisch oder bicyclisch sein kann,
R⁶ ausgewählt ist aus der Gruppe bestehend aus Wasserstoff, Halogen, Hydroxy und C₁₋₆-Alkyl,
R⁷ ausgewählt ist aus der Gruppe bestehend aus Wasserstoff, Halogen, Hydroxy und C₁₋₆-Alkyl,
R^{×} Wasserstoff oder C₁₋₆-Alkyl ist, das gegebenenfalls substituiert ist mit einem bis drei Substituenten, ausgewählt aus der Gruppe bestehend aus Halogen und Hydroxy,
oder ein pharmazeutisch annehmbares Salz davon.

5. Die Verbindung nach einem der Ansprüche 1 bis 4, wobei R¹ Halogen ist und R² Halogen ist, oder ein pharmazeutisch annehmbares Salz davon.

6. Die Verbindung nach einem der Ansprüche 1, 2, 3 oder 5, wobei R³ Wasserstoff ist und R⁴ Wasserstoff ist, oder ein pharmazeutisch annehmbares Salz davon.

7. Die Verbindung nach einem der Ansprüche 1 bis 6, wobei R⁵ ausgewählt ist aus der Gruppe bestehend aus
(a) Phenyl, das gegebenenfalls substituiert ist mit einem bis drei Substituenten, ausgewählt aus der Gruppe bestehend aus Halogen, Cyano, Methyl, Methoxy, Difluormethoxy, Trifluormethoxy und Trifluormethyl,
(b) Cyclopropyl, Cyclobutyl, Cyclopentyl oder Cyclohexyl, wobei das Cyclopropyl gegebenenfalls substituiert ist mit einem bis vier Substituenten, ausgewählt aus der Gruppe bestehend aus Methyl und Halogen,
(c) Benzodioxyl, Pyridinyl, Pyridazinyl, Pyrimidinyl, Thiophenyl, Furanyl, Thiazolyl, Pyrrolyl, Benzofuranyl, Pyrazolyl oder Isoxazolyl, wobei die Pyridinyl-, Pyrazolyl-, Pyrrolyl- und Isoxazolyl-Gruppen gegebenenfalls substituiert sind mit einem oder zwei Substituenten, ausgewählt aus der Gruppe bestehend aus Halogen, Methyl und Trifluormethyl, und
(d) Tetrahydropyranyl oder Oxabicyclohexanyl,
oder ein pharmazeutisch annehmbares Salz davon.

8. Die Verbindung nach einem der Ansprüche 1 bis 7, wobei R⁵ Phenyl ist, das gegebenenfalls substituiert ist mit einem bis drei Substituenten, ausgewählt aus der Gruppe bestehend aus Halogen, Cyano, Methyl, Methoxy, Difluormethoxy, Trifluormethoxy und Trifluormethyl, oder ein pharmazeutisch annehmbares Salz davon.

9. Die Verbindung nach Anspruch 1, ausgewählt aus: 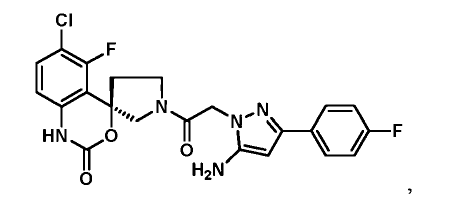 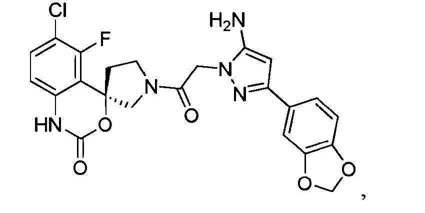 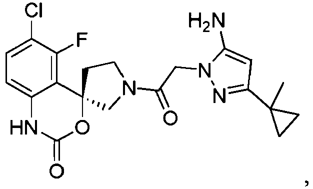 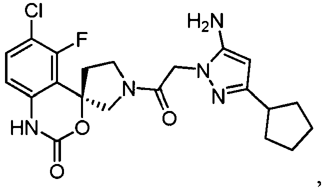 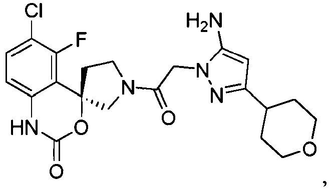 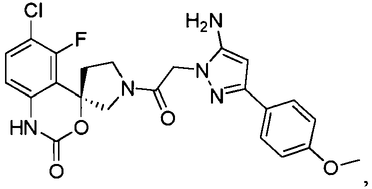 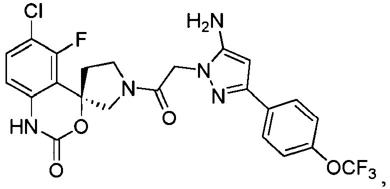 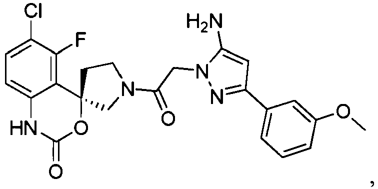 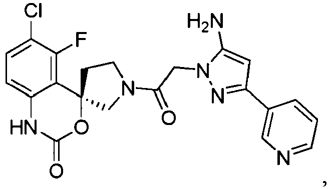 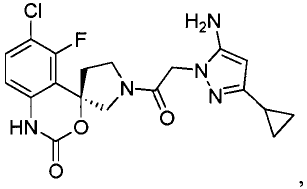 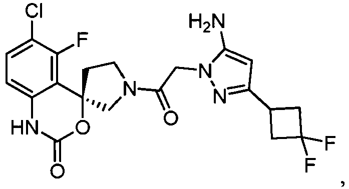 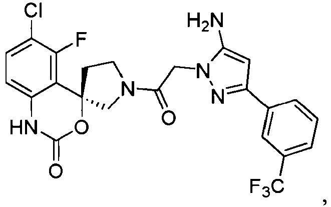 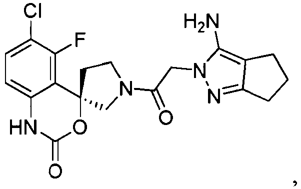 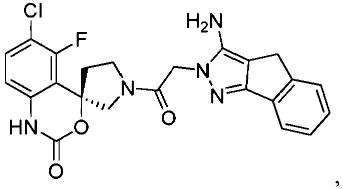 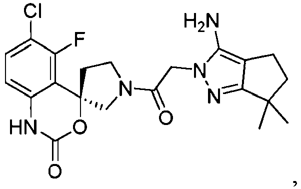 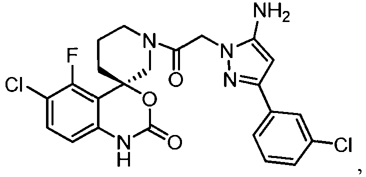 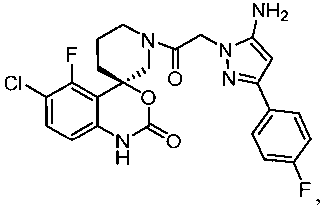 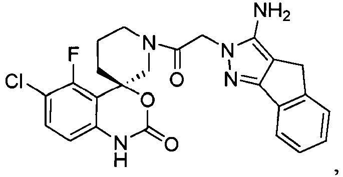 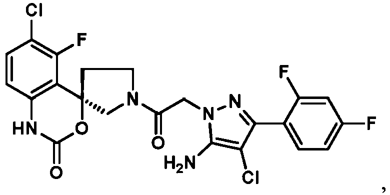 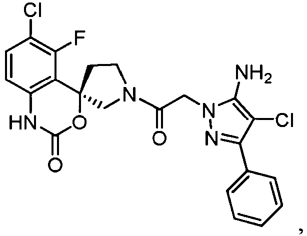 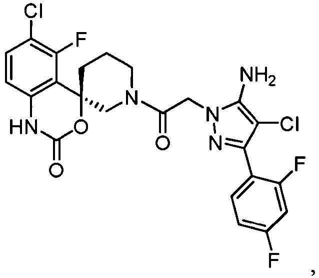 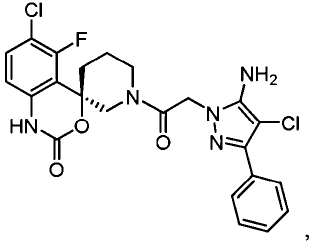 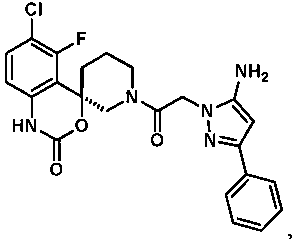 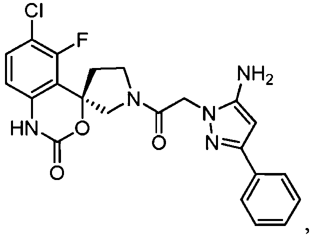 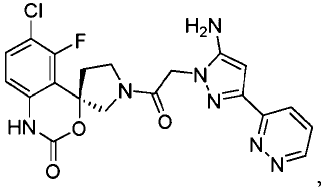 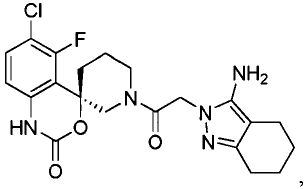 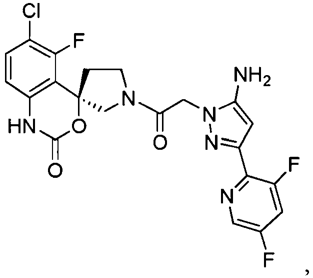 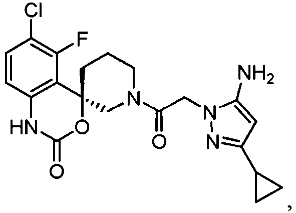 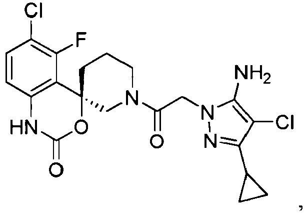 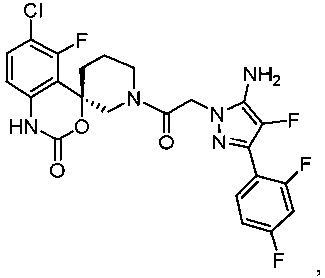 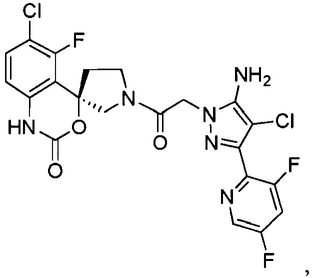 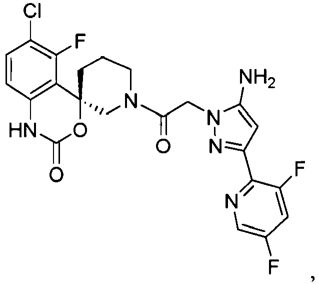 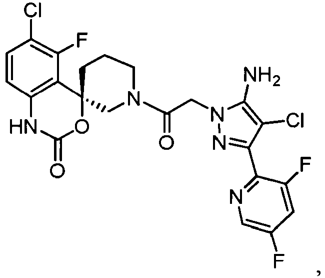 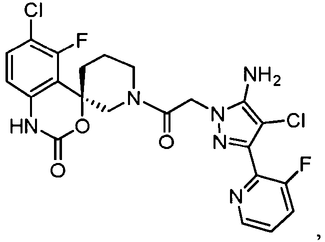 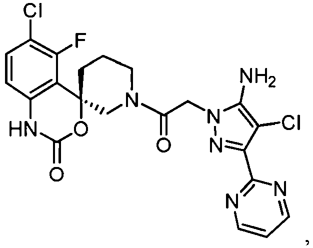 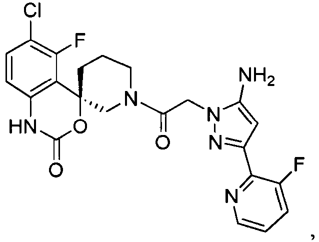 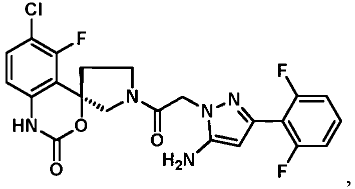 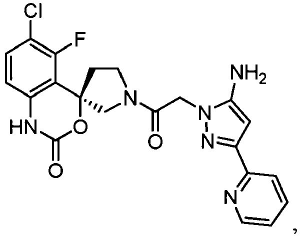 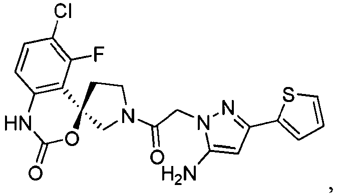 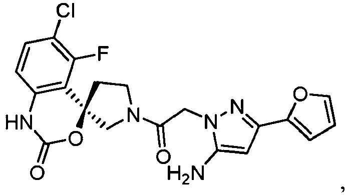 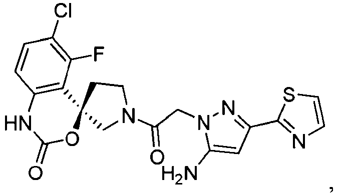 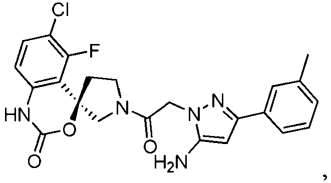 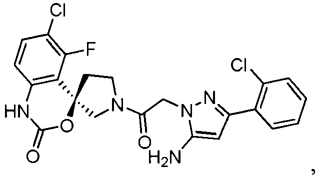 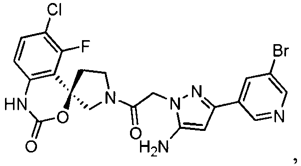 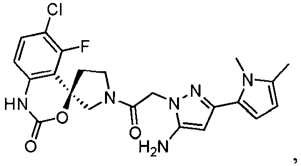 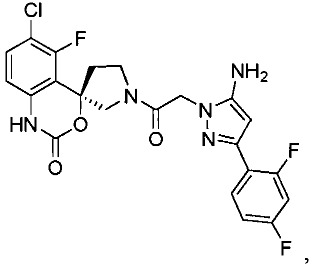 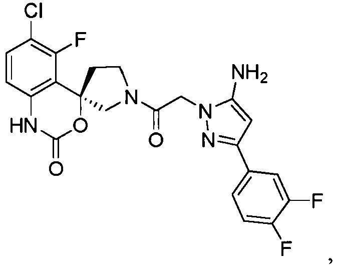 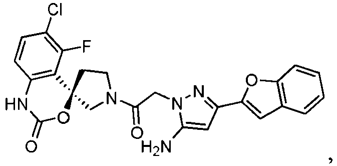 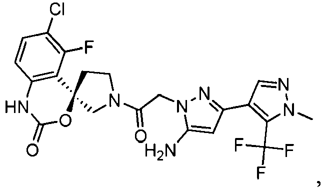 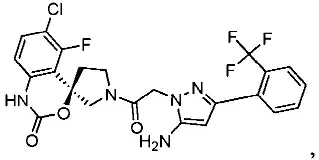 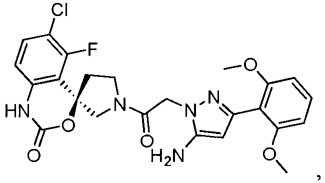 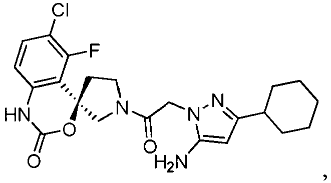 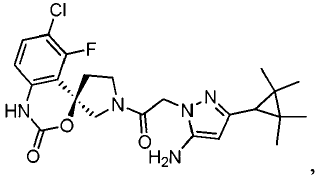 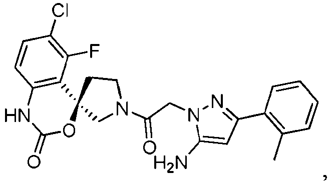 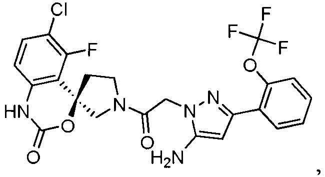 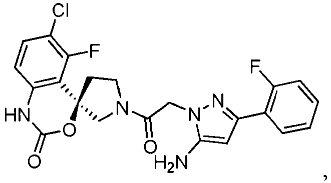 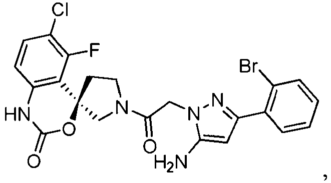 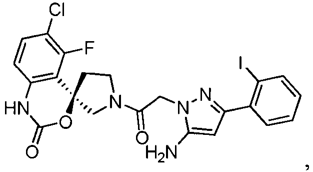 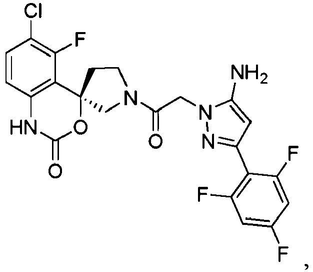 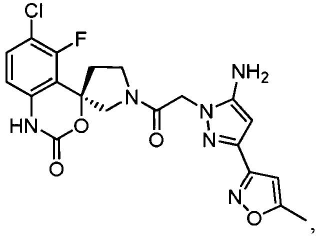 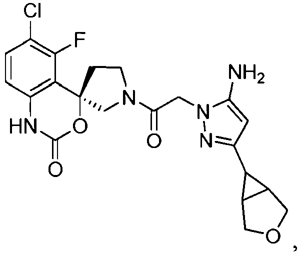 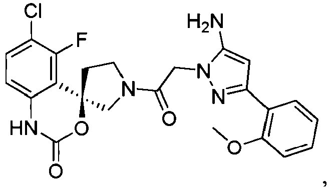 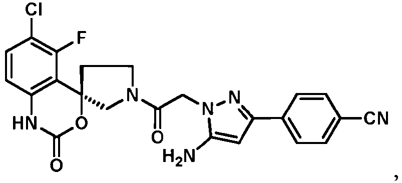 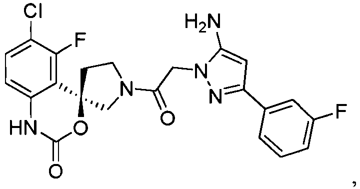 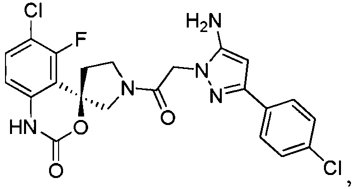 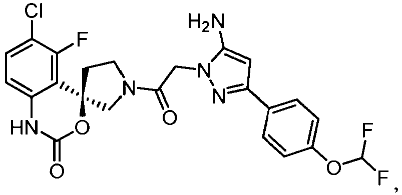 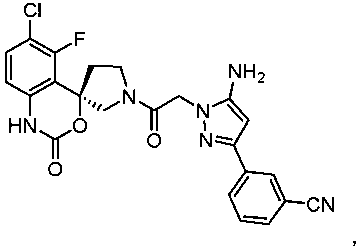 oder ein pharmazeutisch annehmbares Salz davon.

10. Eine pharmazeutische Zusammensetzung, die eine Verbindung nach einem der Ansprüche 1 bis 9 oder ein pharmazeutisch annehmbares Salz davon und einen pharmazeutisch annehmbaren Träger umfasst.

11. Eine Verbindung nach einem der Ansprüche 1 bis 9 oder ein pharmazeutisch annehmbares Salz davon oder eine pharmazeutische Zusammensetzung nach Anspruch 10 zur Verwendung bei einem Verfahren zur Behandlung von Sehstörungen, diabetischer Retinopathie, diabetischem Makulaödem, Netzhautvenenverschluss, hereditärem Angioödem, Diabetes, Pankreatitis, Hirnblutung, Nephropathie, Kardiomyopathie, Neuropathie, entzündlicher Darmerkrankung, Arthritis, Entzündungen, septischem Schock, Hypotonie, Krebs, Atemnotsyndrom bei Erwachsenen, disseminierter intravasaler Koagulopathie, Blutgerinnung während einer Herz-Lungen-Bypass-Operation, Blutungen nach einer Operation, Uveitis, posteriorer Uveitis oder feuchter altersbedingter Makuladegeneration bei einem Säuger.

12. Die Verbindung gemäß einem der Ansprüche 1 bis 9 oder ein pharmazeutisch annehmbares Salz davon zur Verwendung in der Therapie.

## Revendications

1. Composé de Formule I : 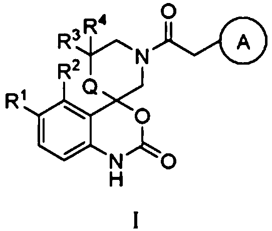 dans lequel 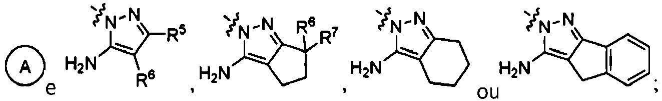
Q est -CH₂- ou absent ;
R¹ est choisi dans le groupe consistant en un hydrogène, un halo, un hydroxy et un alkyle en C₁₋₆ ;
R² est choisi dans le groupe consistant en un hydrogène, un halo, un hydroxy et un alkyle en C₁₋₆ ;
R³ est choisi dans le groupe consistant en un hydrogène, un halo, un hydroxy et un alkyle en C₁₋₆ ;
R⁴ est choisi dans le groupe consistant en un hydrogène, un halo, un hydroxy et un alkyle en C₁₋₆ ;
R⁵ est choisi dans le groupe consistant en
(a) un phényle, qui est éventuellement substitué par un à trois substituants choisis dans le groupe consistant en un halo, un cyano, R^{X} et OR^{X} ;
(b) un cycloalkyle en C₃₋₆, qui est éventuellement substitué par un à quatre substituants choisis dans le groupe consistant en un halo et R^{X} ;
(c) un hétéroaryle, qui peut être monocyclique ou bicyclique, qui est éventuellement substitué par un ou deux substituants choisis dans le groupe consistant en un halo et R^{X} ; et
(d) un hétérocyclyle, qui peut être monocyclique ou bicyclique ;
R⁶ est choisi dans le groupe consistant en un hydrogène, un halo, un hydroxy et un alkyle en C₁₋₆ ;
R⁷ est choisi dans le groupe consistant en un hydrogène, un halo, un hydroxy et un alkyle en C₁₋₆ ;
R^{X} est un hydrogène ou un alkyle en C₁₋₆, qui est éventuellement substitué par un à trois substituants choisis dans le groupe consistant en un halo et un hydroxy ;
ou sel pharmaceutiquement acceptable de celui-ci.

2. Composé selon la revendication 1, dans lequel Q est -CH₂-, ou sel pharmaceutiquement acceptable de celui-ci.

3. Composé selon les revendications 1 ou 2 de Formule la : 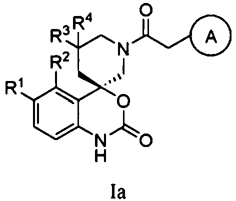 dans lequel 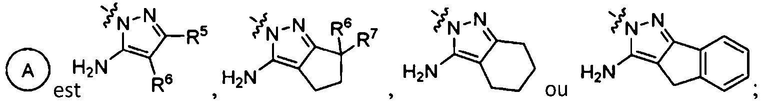
R¹ est choisi dans le groupe consistant en un hydrogène, un halo, un hydroxy et un alkyle en C₁₋₆ ;
R² est choisi dans le groupe consistant en un hydrogène, un halo, un hydroxy et un alkyle en C₁₋₆ ;
R³ est choisi dans le groupe consistant en un hydrogène, un halo, un hydroxy et un alkyle en C₁₋₆ ;
R⁴ est choisi dans le groupe consistant en un hydrogène, un halo, un hydroxy et un alkyle en C₁₋₆ ;
R⁵ est choisi dans le groupe consistant en
(a) un phényle, qui est éventuellement substitué par un à trois substituants choisis dans le groupe consistant en un halo, un cyano, R^{X} et OR^{X} ;
(b) un cycloalkyle en C₃₋₆, qui est éventuellement substitué par un à quatre substituants choisis dans le groupe consistant en un halo et R^{X} ;
(c) un hétéroaryle, qui peut être monocyclique ou bicyclique, qui est éventuellement substitué par un ou deux substituants choisis dans le groupe consistant en un halo et R^{X} ; et
(d) un hétérocyclyle, qui peut être monocyclique ou bicyclique ;
R⁶ est choisi dans le groupe consistant en un hydrogène, un halo, un hydroxy et un alkyle en C₁₋₆ ;
R⁷ est choisi dans le groupe consistant en un hydrogène, un halo, un hydroxy et un alkyle en C₁₋₆ ;
R^{X} est un hydrogène ou un alkyle en C₁₋₆, qui est éventuellement substitué par un à trois substituants choisis dans le groupe consistant en un halo et un hydroxy ;
ou sel pharmaceutiquement acceptable de celui-ci.

4. Composé selon la revendication 1 de Formule 1b 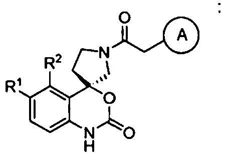 dans lequel 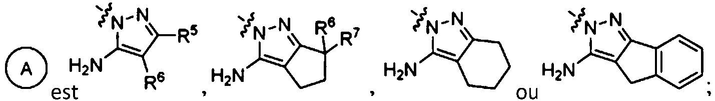
R¹ est choisi dans le groupe consistant en un hydrogène, un halo, un hydroxy et un alkyle en C₁₋₆ ;
R² est choisi dans le groupe consistant en un hydrogène, un halo, un hydroxy et un alkyle en C₁₋₆ ;
R⁵ est choisi dans le groupe consistant en
(a) un phényle, qui est éventuellement substitué par un à trois substituants choisis dans le groupe consistant en un halo, un cyano, R^{X} et OR^{X} ;
(b) un cycloalkyle en C₃₋₆, qui est éventuellement substitué par un à quatre substituants choisis dans le groupe consistant en un halo et R^{X} ;
(c) un hétéroaryle, qui peut être monocyclique ou bicyclique, qui est éventuellement substitué par un ou deux substituants choisis dans le groupe consistant en un halo et R^{X} ; et
(d) un hétérocyclyle, qui peut être monocyclique ou bicyclique ;
R⁶ est choisi dans le groupe consistant en un hydrogène, un halo, un hydroxy et un alkyle en C₁₋₆ ;
R⁷ est choisi dans le groupe consistant en un hydrogène, un halo, un hydroxy et un alkyle en C₁₋₆ ;
R^{X} est un hydrogène ou un alkyle en C₁₋₆, qui est éventuellement substitué par un à trois substituants choisis dans le groupe consistant en un halo et un hydroxy,
ou sel pharmaceutiquement acceptable de celui-ci.

5. Composé selon l'une quelconque des revendications 1 à 4, dans lequel R¹ est un halo et R² est un halo, ou sel pharmaceutiquement acceptable de celui-ci.

6. Composé selon l'une quelconque des revendications 1, 2, 3 ou 5, dans lequel R³ est un hydrogène et R⁴ est un hydrogène, ou sel pharmaceutiquement acceptable de celui-ci.

7. Composé selon l'une quelconque des revendications 1 à 6, dans lequel R⁵ est choisi dans le groupe consistant en
(a) un phényle, qui est éventuellement substitué par un à trois substituants choisis dans le groupe consistant en un halo, un cyano, un méthyle, un méthoxy, un difluorométhoxy, un trifluorométhoxy et un trifluorométhyle ;
(b) un cyclopropyle, un cyclobutyle, un cyclopentyle ou un cyclohexyle, dans lequel ledit cyclopropyle est éventuellement substitué par un à quatre substituants choisis dans le groupe consistant en un méthyle et un halo ;
(c) un benzodioxyle, un pyridinyle, un pyridazinyle, un pyrimidinyle, un thiophényle, un furanyle, un thiazolyle, un pyrrolyle, un benzofuranyle, un pyrazolyle ou un isoxazolyle, dans lequel lesdits groupes pyridinyle, pyrazolyle, pyrrolyle et isoxazolyle sont éventuellement sustitiés par un ou deux substituants choisis dans le groupe consistant en un halo, un méthyle et un trifluorométhyle; et
(d) un tétrahydropyranyle ou un oxabicyclohexanyle;
ou sel pharmaceutiquement acceptable de celui-ci.

8. Composé selon l'une quelconque des revendications 1 à 7, dans lequel R⁵ est un phényle, qui est éventuellement substitué par un à trois substituants choisis dans le groupe consistant en un halo, un cyano, un méthyle, un méthoxy, un difluorométhoxy, un trifluorométhoxy et un trifluorométhyle; uo sel pharmaceutiquement acceptable de celuli-ci.

9. Composé selon la revendication 1 choisi parmi: 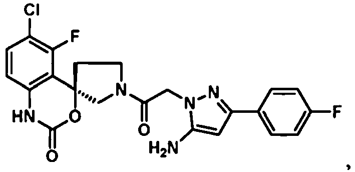 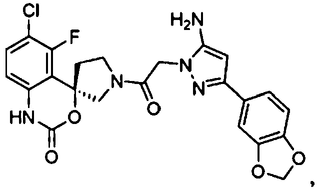 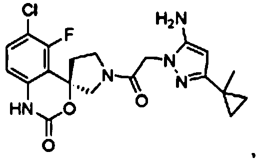 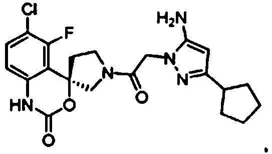 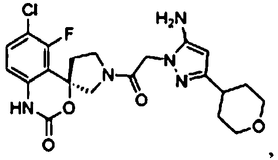 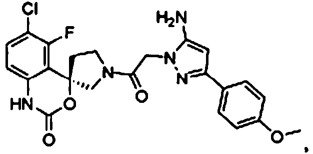 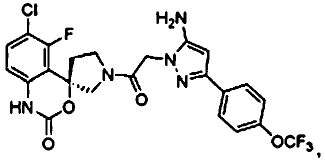 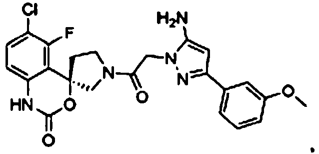 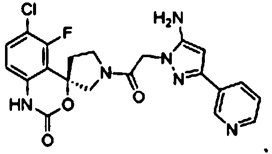 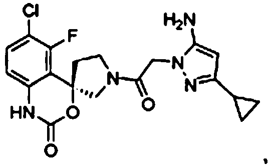 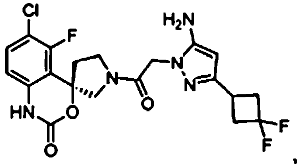 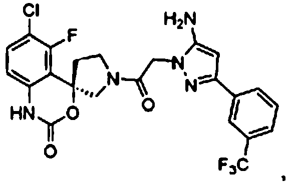 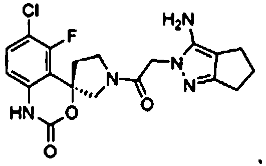 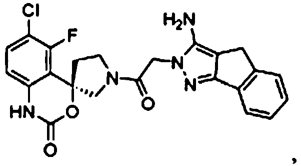 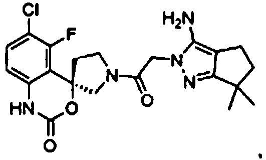 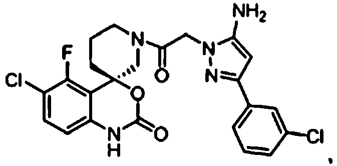 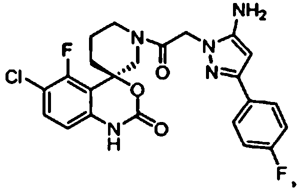 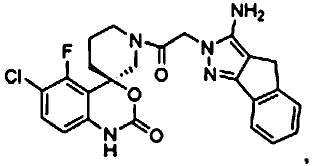 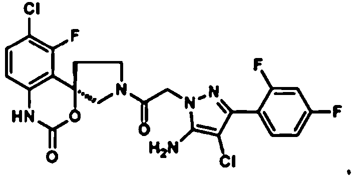 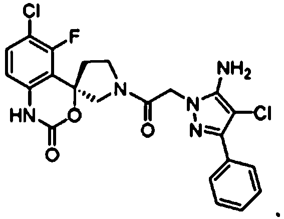 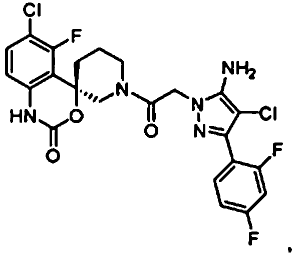 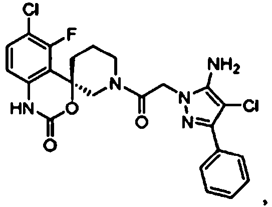 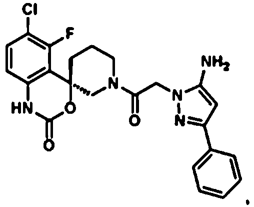 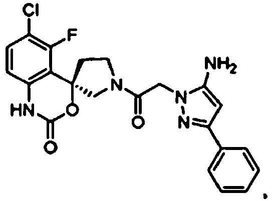 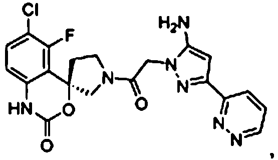 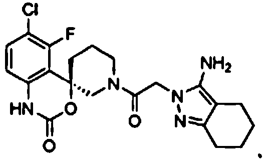 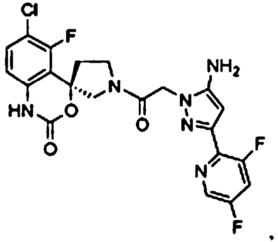 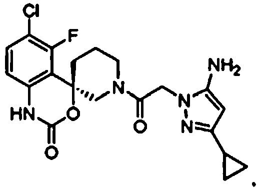 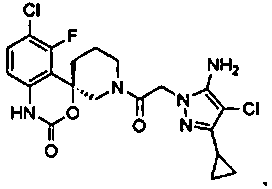 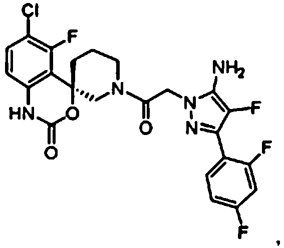 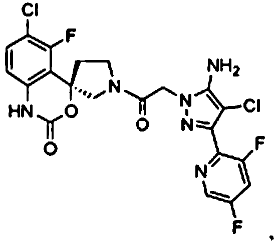 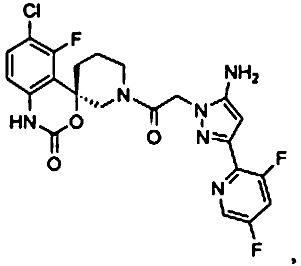 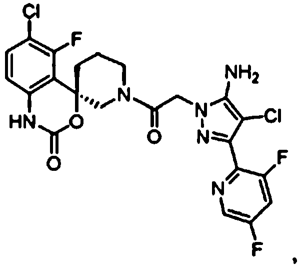 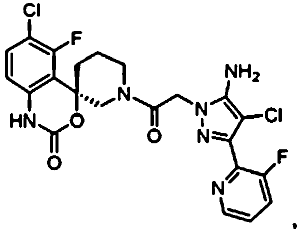 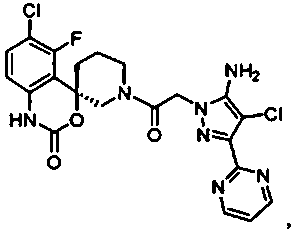 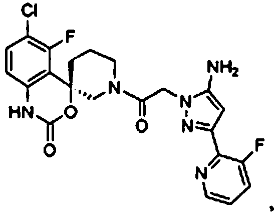 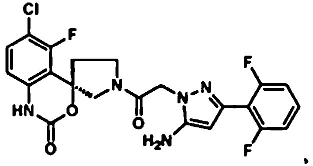 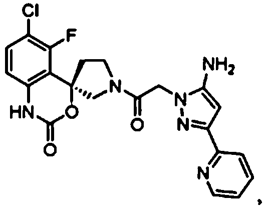 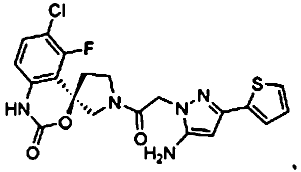 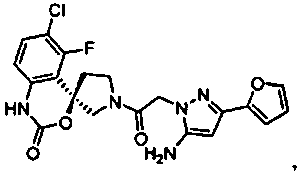 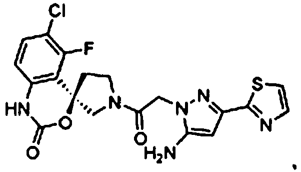 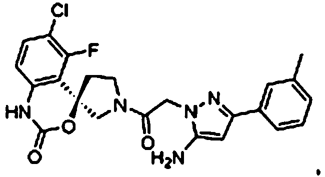 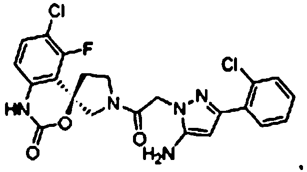 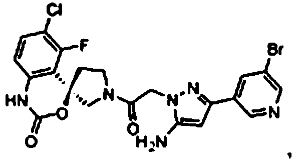 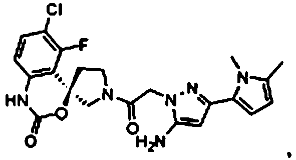 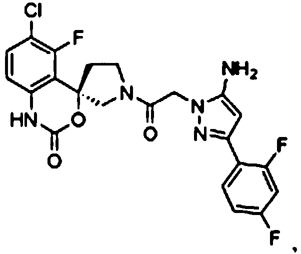 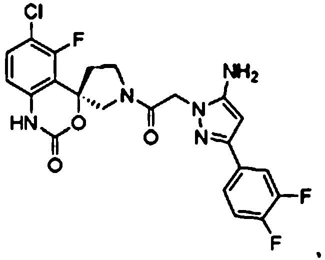 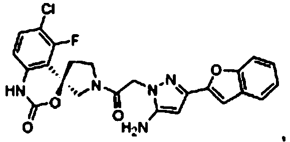 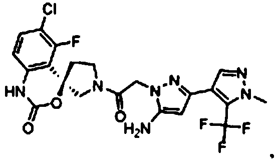 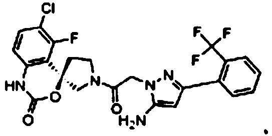 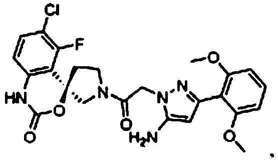 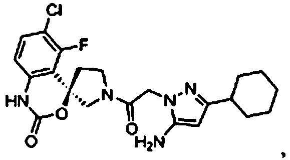 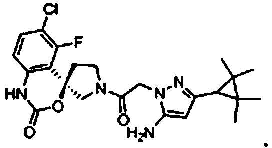 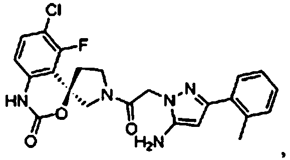 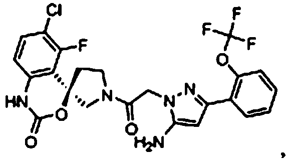 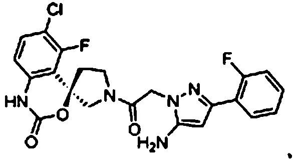 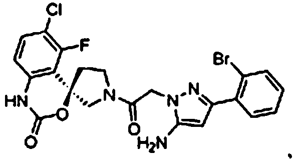 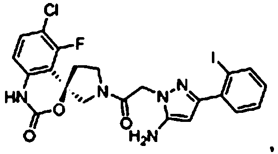 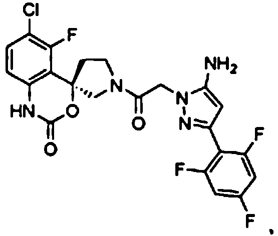 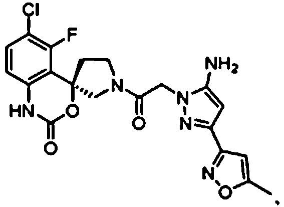 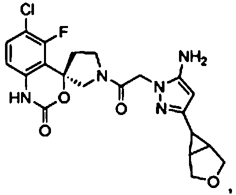 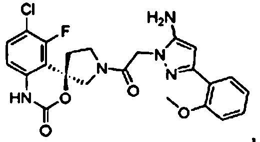 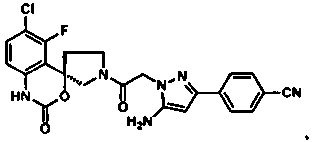 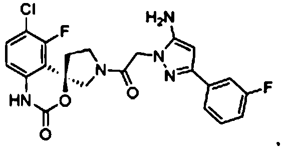 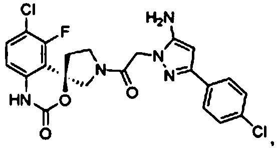 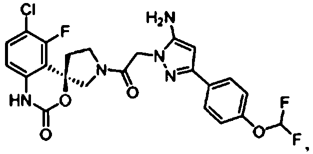 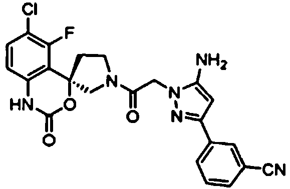 ou sel pharmaceutiquement acceptable de celui-ci.

10. Composition pharmaceutique comprenant un composé selon l'une quelconque des revendications 1 à 9 ou un sel pharmaceutiquement acceptable de celui-ci et un support pharmaceutiquement acceptable.

11. Composé selon l'une quelconque des revendications 1 à 9, ou sel pharmaceutiquement acceptable de celui-ci, ou composition pharmaceutique selon la revendication 10, destiné(e) à être utilisé(e) dans un procédé de traitement d'une activité visuelle altérée, d'une rétinopathie diabétique, d'un œdème maculaire diabétique, d'une occlusion veineuse rétinienne, d'un angio-œdème héréditaire, du diabète, d'une pancréatite, d'une hémorragie cérébrale, d'une néphropathie, d'une cardiomyopathie, d'une neuropathie, d'une maladie inflammatoire chronique de l'intestin, de l'arthrite, de l'inflammation, du choc septique, de l'hypotension, du cancer, du syndrome de détresse respiratoire de l'adulte, de la coagulation intravasculaire disséminée, de la coagulation sanguine pendant un pontage cardiopulmonaire, d'un saignement dû à une chirurgie postopératoire, d'une uvéite, d'une uvéite postérieure ou d'une dégénérescence maculaire humide liée à l'âge chez un mammifère.

12. Composé selon l'une quelconque des revendications 1 à 9, ou sel pharmaceutiquement acceptable de celui-ci, destiné à être utilisé en thérapie.
